# EUROPÄISCHE PATENTSCHRIFT

(11) **EP 2 827 830 B1**
(45) Veröffentlichungstag und Bekanntmachung des Hinweises auf die Patenterteilung: **09.08.2017**
(21) Anmeldenummer: 12781315.2
(22) Anmeldetag: 05.11.2012
(51) Int. Cl.: A61K 8/44, A61K 8/34, A61Q 19/00, A01N 47/44

(54) **KONSERVIERUNGSMITTELZUSAMMENSETZUNGEN UND KOSMETIKA ENTHALTEND DIESE**
PRESERVATIVE COMPOSITION AND COSMETIC PRODUCTS CONTAINING SAME
COMPOSITIONS D'AGENTS CONSERVATEURS ET PRODUITS COSMÉTIQUES LES CONTENANT

(30) Priorität: 21.12.2011 DE 102011089407
(43) Veröffentlichungstag der Anmeldung: 28.01.2015
(73) Patentinhaber: Henkel AG & Co. KGaA, 40589 Düsseldorf (DE)
(72) Erfinder: HARTMANN, Ira, 40589 Düsseldorf (DE); WALDMANN-LAUE, Marianne, 40789 Monheim (DE); HEINEN, Soraya, 50858 Köln (DE)
(86) Internationale Anmeldenummer: PCT/EP2012/071828
(87) Internationale Veröffentlichungsnummer: WO 2013/091978

(56) Entgegenhaltungen:
- EP-B1- 1 414 394
- FR-A1- 2 921 560
- US-A1- 2009 163 445
- US-A1- 2010 092 420
- US-A1- 2010 284 942
- KLEIN K: "HUMECTANTS: MORE THAN MEETS THE EYE (OR SKIN)", COSMETICS & TOILETRIES, WHEATON, IL, US, Bd. 120, Nr. 2, 1. Januar 2005 (2005-01-01), Seite 30/31, XP009068520, ISSN: 0361-4387

## Beschreibung

Die vorliegende Erfindung betrifft neue Konservierungsmittelgemische und deren Einsatz auf dem Gebiet der Kosmetik.

Durch ihre Zusammensetzung können kosmetische Mittel als Nährmedium für Keime und Mikroorganismen dienen. Diese Keime können einerseits eine mikrobielle Kontamination des Verbrauchers herbeiführen, andererseits können sie Inhaltsstoffe der Kosmetika verändern und dabei Stoffe mit unerwünschten Wirkungen wie Sensibilisierung oder Hautreizung bilden. Um diese unerwünschten Folgen zu verhindern und um eine bestimmte Mindesthaltbarkeit der Kosmetika zu gewährleisten, müssen diese konserviert werden. Da Konservierungsmittel ihrerseits ein irritatives Potential besitzen, ist ihr Einsatz in Kosmetika streng reglementiert.

Die Hautmikroflora hat einen entscheidenden Einfluß auf unterschiedliche kosmetische Parameter. So spielen pathogene Keime wie Staphylococcus aureus eine entscheidende Rolle bei der Entstehung von Hautunreinheiten. Neueste Studien deuten auch darauf hin, dass eine unausgeglichene Hautmikroflora einen Einfluß auf die Hautalterung ausüben kann, da unerwünschte Keime zu einer gesteigerten Immunabwehr der Haut führen, die ihrerseits zu vermehrten Entzündungsreaktionen führt, in deren Verlauf Hautalterungsmarker stimuliert werden.

Es besteht daher nach wie vor der Bedarf nach Konservierungsmittelzusammensetzungen, die einerseits die Besiedlung des Produktes oder der Haut mit unerwünschten Keimen verhindern und andererseits die natürliche Hautflora nicht oder nicht wesentlich beeinträchtigen.

Die Mischung verschiedener antimkrobieller Substanzen zur Steigerung der antimikrobiellen Aktivität ist grundsätzlich bekannt. So schlägt die WO 03/043 593 A1 vor, herkömmliche antibakterielle Substanzen wie Triclosan, Phenoxyethanol oder Hexetidin mit Ethyllauroylarginat zu kombinieren, um die antibakterielle Wirkung zu verstärken. In der WO 2007/014580 A1 werden Konservierungsmittelgemische vorgeschlagen, die neben Ethyllauroylarginat Salze von organischen oder anorganischen Säuren, insbesondere Natriumcitrat, Natriumacetat, Natriumglutamat, Natriumfumarat, Natriummalat, Natriumgluconat, Natriumlaurat, Natriumlactat, Natriumhexametaphosphat, Natrium-tert-Butyl-hydrochinat, Natriumpropylparabenat oder die Hydrochloride von Glucosamin oder Ethanolamin enthalten. Kosmetische Zusammensetzungen, die ein Konservierungsmittelgemisch aus Ethyllauroylarginat und Parabenen, Imidzolylharnstoff, Phenoxyethanol, DMDM Hydantoin, 2-Methyl-5-chlor-3,4-isothiazolinon/2-Methyl-3,4-isothiazolinon und Quaternium-15 enthalten, werden in der EP 1414394 B1 offenbart.

Es besteht weiter der Bedarf, antimikrobielle Zusammensetzungen bereitzustellen, die bei geringer Einsatzmenge hocheffektiv sind. Der vorliegenden Erfindung lag die Aufgabe zugrunde, synergistische Wirkstoffkombinationen aufzufinden, die in geringen Konzentrationen hochwirksam sind und aufgrund insgesamt verringerter Einsatzmengen die Herstellung reiz- und sensibilisierungsarmer Kosmetika ermöglichen.

Eine weitere Aufgabe war insbesondere, wirksame Konservierungsmittel zu finden, die den Verzicht auf 4-Hydroxybenzoesäure und 4-Hydroxybenzoesäureester, so genannte "Parabene", ermöglichen.

Ein erster Gegenstand der vorliegenden Erfindung sind Konservierungsmittelzusammensetzungen, enthaltend
a) mindestens eine Verbindung der Formel (I) 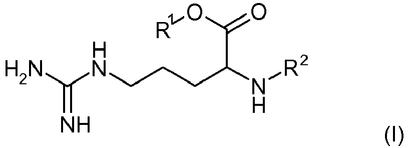 worin
   R¹ eine lineare oder verzweigte (C₁ bis C₁₈)-Alkylgruppe (insbesondere eine (C₁ bis C₃)-alkylgruppe) bedeutet und
   R² für eine lineare oder verzweigte (C₈ bis C₁₄)-Acylgruppe steht, und
b) mindestens einen Monoglycerylether der Formel (II) 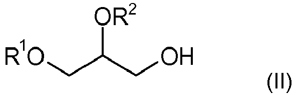 wobei ein Rest der Reste R¹ und R² für einen verzweigten oder linearen, (C₃ bis C₁₂)-Alkylgruppe und der andere dieser Reste für ein Wasserstoffatom steht.

Die erfindungsgemäßen Zusammensetzungen enthalten als ersten Inhaltsstoff der Formel (I) bevorzugt Ethyllauroylarginat (ELA bzw. LAE), auch als Ethyl-N^{alpha}-dodecanoyl-arginat bezeichnet. Jedes Stereoisomere des Ethyllauroylarginats allein oder in Kombination ist erfindungsgemäß, bevorzugt eignet sich jedoch die L-Form. Ethyllauroylarginat ist beispielsweise als Hydrochlorid kommerziell erhältlich 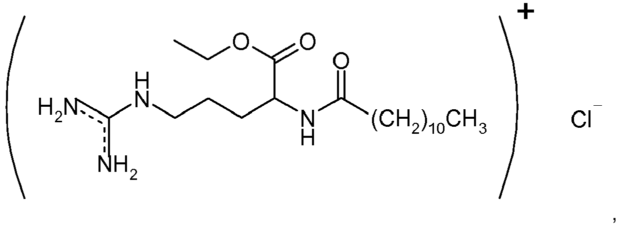 das auch als Ethyl-N^{alpha}-dodecanoyl-L-arginathydrochlorid bezeichnet wird. LAE gilt als unbedenklich, auch für den Einsatz in Nahrungsmitteln.

In einer bevorzugten Ausführungsform enthalten die erfindungsgemäßen Konservierungsmittel-Zusammensetzungen 4-Hydroxybenzoesäure und/oder 4-Hydroxybenzoesäureester in einer Gesamtmenge von null bis maximal 0,1 Gew.-%, bevorzugt null bis 0,05 Gew.-%, besonders bevorzugt null bis 0,00001 Gew.-%, bezogen auf das Gewicht der gesamten Konservierungsmittel-Zusammensetzung.

Als zweiter zwingender Inhaltsstoff ist mindestens ein (C₃ bis C₁₂)-Monoglycerylether der Formel (I) enthalten. Die oben definierte Alkylgruppe des Rests R¹ oder R² des Monoglycerylethers der Formel (I) ist bevorzugt eine verzweigte oder lineare (C₆ bis C₁₂)-Alkylgruppe.

Die oben definierte Alkylgruppe des Monoglycerylethers der Formel (II) ist bevorzugt verzweigt, besonders bevorzugt eine verzweigte (C₆ bis C₁₂)-Alkylgruppe.

Beispiele bevorzugter Monoglycerylether sind 3-(2-Ethylhexyloxy)propan-1,2-diol (in Formel (II) R¹=2-Ethylhexyl, R²=Wasserstoff), 2-(2-Ethylhexyloxy)propan-1,3-diol (in Formel (II) R¹ = Wasserstoff, R² = 2-Ethylhexyl), 3-Propyloxypropan-1,2-diol (in Formel (II) R¹=Propyl, R²=Wasserstoff), 2-Propyloxypropan-1,3-diol (in Formel (II) R¹ = Wasserstoff, R² = Propyl), 3-Octyloxypropan-1,2-diol (in Formel (II) R¹=Octyl, R²=Wasserstoff), 2-Octyloxypropan-1,3-diol (in Formel (II) R¹ = Wasserstoff, R² = Octyl), 3-Decyloxypropan-1,2-diol (in Formel (II) R¹=Decyl, R²=Wasserstoff), 2-Decyloxy-propan-1,3-diol (in Formel (II) R¹ = Wasserstoff, R² = Decyl), 3-Dodecyloxypropan-1,2-diol (in Formel (II) R¹=Dodecyl, R²=Wasserstoff), 2-Dodecyloxypropan-1,3-diol (in Formel (II) R¹ = Wasserstoff, R² = Dodecyl). 3-(2-Ethylhexyloxy)propan-1,2-diol (CAS Nummer: 70445-33-9) ist ganz besonders bevorzugt und wird beispielsweise unter der INCI-Bezeichnung Ethylhexyloxyglycerin z.B. als Sensiva^{®} SC 50 von der Firma Schülke & Mayr vertrieben.

Die Konservierungsmittelzusammensetzungen enthalten die Verbindungen der Formel (I) und der Formel (II) in einem bevorzugten Gewichtsverhältnisbereich der Verbindungen der Formel (I) zu Verbindungen der Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten.

Die Konservierungsmittelzusammensetzungen enthalten besonders bevorzugt Ethyllauroylarginat und 3-(2-Ethylhexyloxy)propan-1,2-diol in einem Gewichtsverhältnisbereich der Verbindungen Ethyllauroylarginat zu 3-(2-Ethylhexyloxy)propan-1,2-diol von 1 zu 10 bis 5 zu 1, besonders bevorzugt von 1 zu 6 bis 3 zu 1, ganz besonders bevorzugt von 1 zu 5 bis 1,5 zu 1.

Es ist erfindungsgemäß günstig neben den Verbindungen der Formel (I) und der Formel (II) zusätzlich in der erfindungsgemäßen Konservierungsmittelzusammensetzung
c) mindestens eine Verbindung aus der Gruppe, die gebildet wird aus
   a. Diolen der Formel (III)

      HO-CH₂-CHR³-(CH₂)ₙ-R⁴ (III),

      in der R³ und R⁴ für -H oder -OH und n = für eine ganze Zahl von 1 bis 10 stehen, wobei R³ und R⁴ stets verschieden sind,
   b. Salzen aromatischer Säuren der Formel (IV) 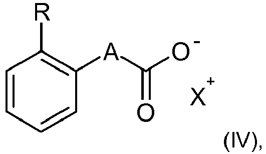 in der R für -H, -OH, ein Halogenatom oder eine C₁-C₄-Alkylgruppe, A für eine direkte Bindung oder Vinylen und X⁺ für ein Alkalimetallion oder den n-ten Teil eines n-wertigen Metallkations steht,
   c. Dehydracetsäure (Va) und/oder deren Salze (Vb) 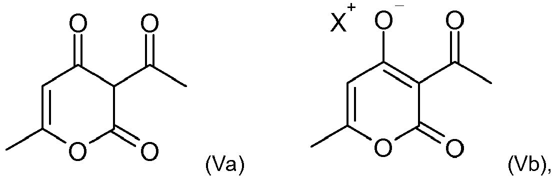 in der X⁺ für ein Alkalimetallion oder den n-ten Teil eines n-wertigen Metallkations steht,
   d. Sorbinsäure und deren Salze,
   e. 2-Phenoxyethanol,
   f. Benzylalkohol, einzusetzen.

Geeignet als zusätzliche Komponente c)a sind Diole der Formel (III),

HO-CH₂-CHR³-(CH₂)ₙ-R⁴ (III),

in der R³ und R⁴ für -H oder -OH und n = für eine ganze Zahl von 1 bis 10 stehen, wobei R³ und R⁴ stets verschieden sind. Es handelt sich dabei entweder um 1,2-Diole oder um alpha,omega-Diole mit 3 bis 12 Kohlenstoffatomen. Erfindungsgemäß geeignet sind beispielsweise 1,2-Propandiol, 1,2-Butandiol, 1,2-Pentandiol, 1,2-Hexandiol, 1,2-Heptandiol, 1,2-Octandiol, 1,2-Nonandiol, 1,2-Decandiol, 1,2-Undecandiol, 1,2-Dodecandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol sowie deren Mischungen.

Besonders bevorzugte erfindungsgemäße Konservierungsmittelzusammensetzungen enthalten neben der erfindungsgemäßen Wirkstoffkombination zusätzlich mindestens eine weitere Verbindung aus der Gruppe
- 1,2-Propandiol (R³ = -OH, R⁴ = -H und n= 1 in Formel (III)) und/oder
- 1,3-Propandiol (R³ = -H, R⁴ = -OH und n= 1 in Formel (III)) und/oder
- 1,2-Hexandiol (R³ = -OH, R⁴ = -H und n= 4 in Formel (III)) und/oder
- 1,6-Hexandiol (R³ = -H, R⁴ = -OH und n= 4 in Formel (III)) und/oder
- 1,2-Octandiol (R³ = -OH, R⁴ = -H und n= 6 in Formel (III)).

Bevorzugt sind Diole der Formel (III) mit n = 4 sowie n = 6. Besonders bevorzugt sind Diole der Formel (III) mit n = 4, also 1,2-Hexandiol und 1,6-Hexandiol. Ein außerordentlich bevorzugtes Diol der Formel (III) ist 1,6-Hexandiol.

Zusätzlich zu dem bzw. den Diol(en) der Formel (III) oder an ihrer Stelle können die erfindungsgemäßen Konservierungsmittelzusammensetzungen als zusätzlich zur erfindungsgemäßen Wirkstoffkombination enthaltenen Komponente c)b mindestens eine weitere Verbindung aus der Gruppe der Salze aromatischer Säuren der Formel (IV) 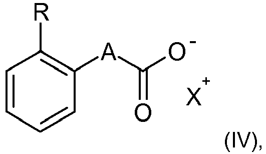 enthalten, in der R für -H, -OH, ein Halogenatom oder eine C₁-C₄-Alkylgruppe, A für eine direkte Bindung oder Vinylen und X⁺ für H₃O⁺, ein Alkalimetallion oder den n-ten Teil eines n-wertigen Metallkations steht.

Erfindungsgemäß geeignet sind beispielsweise die Natrium-, Kalium- oder Zinksalze von Säuren, in denen R für -H, -OH, -Cl, -Br, -CH₃, -CH₂CH₃, -CH(CH₃)₂, -(CH₂)₂CH₃, -C(CH₃)₃, -(CH₂)₃CH₃ steht.

Bevorzugte erfindungsgemäße Konservierungsmittelzusammensetzungen enthalten als zusätzliche Komponente c) mindestens eine weitere Verbindung aus der Gruppe
- Natriumbenzoat (R = -H und A = direkte Bindung und X⁺ = Na+ in Formel (IV)) und/oder
- Natriumsalicylat (R = -OH und A = direkte Bindung und X⁺ = Na+ in Formel (IV)) und/oder
- Kaliumbenzoat (R = -H und A = direkte Bindung und X⁺ = K+ in Formel (IV)) und/oder
- Kaliumsalicylat (R = -OH und A = direkte Bindung und X⁺ = K+ in Formel (IV)) und/oder
- Natriumcinnamat (R = -H und A = Vinylen und X⁺ = Na+ in Formel (IV)) und/oder
- Kaliumcinnamat (R = -H und A = Vinylen und X⁺ = K+ in Formel (IV)) und/oder
- Zimtsäure (R = -H und A = Vinylen und X⁺ = H₃O+ in Formel (IV)).

Zusätzlichen zu dem bzw. den Diol(en) der Formel (III) oder dem/den Salz(en) aromatischer Säuren der Formel (IV) oder an ihrer Stelle können die erfindungsgemäßen Konservierungsmittelzusammensetzungen als zusätzlichen Inhaltsstoff c)c mindestens eine weitere Verbindung aus der Gruppe Dehydracetsäure (Va) und/oder deren Salze (Vb) 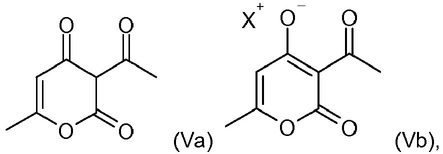 enthalten, in der X⁺ für ein Alkalimetallion oder den n-ten Teil eines n-wertigen Metallkations steht. Hier sind Dehydracetsäure, Kaliumdehydracetat und Natriumdehydracetat besonders bevorzugt.

Zusätzlichen zu dem bzw. den Diol(en) der Formel (III) oder dem/den Salz(en) aromatischer Säuren der Formel (IV) und/oder mindestens einer (einem) Dehydracetsäure(salz) oder an ihrer Stelle können die erfindungsgemäßen Konservierungsmittelzusammensetzungen Sorbinsäure und/oder deren Salz enthalten. Wenn Sorbinsäure und/oder deren Salz enthalten ist, ist es wiederum bevorzugt, Natriumsorbat und/oder Kaliumsorbat zu verwenden.

Die nachfolgenden erfindungsgemäßen Konservierungsmittelzusammensetzungen (A) bis (X) gelten als erfindungsgemäß besonders bevorzugt:
(A): Konservierungsmittel-Zusammensetzungen, enthaltend
   a) mindestens eine Verbindung der Formel (I) 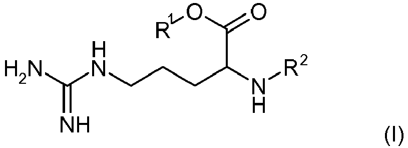 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 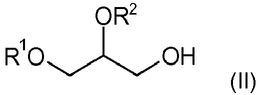 wobei ein Rest der Reste R¹ und R² für einen verzweigten oder linearen, (C₃ bis C₁₂)-Alkylgruppe und der andere dieser Reste für ein Wasserstoffatom steht, und
   c) mindestens ein Diol der Formel (III)

      HO-CH₂-CHR³-(CH₂)ₙ-R⁴ (III),

      in der R³ für ein Wasserstoffatom, R⁴ für eine Gruppe -OH und n = für eine ganze Zahl von 1 bis 10 stehen.
(B): Konservierungsmittel-Zusammensetzungen, enthaltend
   a) mindestens eine Verbindung der Formel (I) 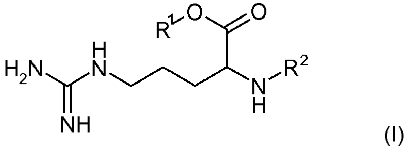 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 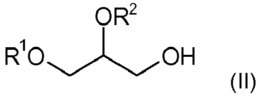 wobei ein Rest der Reste R¹ und R² für einen verzweigten oder linearen, (C₃ bis C₁₂)-Alkylgruppe und der andere dieser Reste für ein Wasserstoffatom steht, und
   c) mindestens ein Diol der Formel (III)

      HO-CH₂-CHR³-(CH₂)ₙ-R⁴ (III),

      in der R³ für eine Gruppe -OH, R⁴ für ein Wasserstoffatom und n = für eine ganze Zahl von 1 bis 10 stehen.
(C): Konservierungsmittel-Zusammensetzungen, enthaltend
   a) Ethyllauroylarginat und
   b) 3-(2-Ethylhexyloxy)propan-1,2-diol und
   c) mindestens ein Diol der Formel (III)

      HO-CH₂-CHR³-(CH₂)ₙ-R⁴ (III),

      in der R³ für ein Wasserstoffatom, R⁴ für eine Gruppe -OH und n = für eine ganze Zahl von 1 bis 10 stehen.
(D): Konservierungsmittel-Zusammensetzungen, enthaltend
   a) Ethyllauroylarginat und
   b) 3-(2-Ethylhexyloxy)propan-1,2-diol und
   c) mindestens ein Diol der Formel (III)

      HO-CH₂-CHR³-(CH₂)ₙ-R⁴ (III),

      in der R³ für eine Gruppe -OH, R⁴ für ein Wasserstoffatom und n = für eine ganze Zahl von 1 bis 10 stehen.
(E): Konservierungsmittel-Zusammensetzungen, enthaltend
   a) Ethyllauroylarginat und
   b) 3-(2-Ethylhexyloxy)propan-1,2-diol und
   c) 1,6-Hexandiol.
(F): Konservierungsmittel-Zusammensetzungen, enthaltend
   a) Ethyllauroylarginat und
   b) 3-(2-Ethylhexyloxy)propan-1,2-diol und
   c) 1,2-Hexandiol.
(G): Konservierungsmittel-Zusammensetzungen, enthaltend
   a) mindestens eine Verbindung der Formel (I) 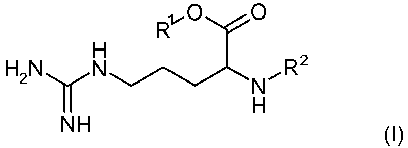 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 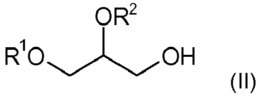 wobei ein Rest der Reste R¹ und R² für einen verzweigten oder linearen, (C₃ bis C₁₂)-Alkylgruppe und der andere dieser Reste für ein Wasserstoffatom steht, und
   c) mindestens ein Diol der Formel (III)

      HO-CH₂-CHR³-(CH₂)ₙ-R⁴ (III),

      in der R³ für ein Wasserstoffatom, R⁴ für eine Gruppe -OH und n = für eine ganze Zahl von 1 bis 10 stehen,
   mit der Massgabe, dass die Verbindungen der Formel (I) und die Verbindungen der Formel (II) in einem Gewichtsverhältnisbereich der Verbindungen der Formel (I) zu Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.
(H): Konservierungsmittel-Zusammensetzungen, enthaltend
   a) mindestens eine Verbindung der Formel (I) 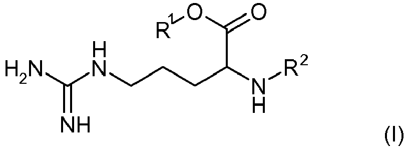 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 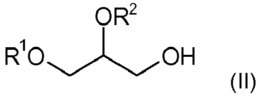 wobei ein Rest der Reste R¹ und R² für einen verzweigten oder linearen, (C₃ bis C₁₂)-Alkylgruppe und der andere dieser Reste für ein Wasserstoffatom steht, und
   c) mindestens ein Diol der Formel (III)

      HO-CH₂-CHR³-(CH₂)ₙ-R⁴ (III),

      in der R³ für eine Gruppe -OH, R⁴ für ein Wasserstoffatom und n = für eine ganze Zahl von 1 bis 10 stehen,
   mit der Massgabe, dass die Verbindungen der Formel (I) und die Verbindungen der Formel (II) in einem Gewichtsverhältnisbereich der Verbindungen der Formel (I) zu Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.
(I): Konservierungsmittel-Zusammensetzungen, enthaltend
   a) Ethyllauroylarginat und
   b) 3-(2-Ethylhexyloxy)propan-1,2-diol und
   c) mindestens ein Diol der Formel (III)

      HO-CH₂-CHR³-(CH₂)ₙ-R⁴ (III),

      in der R³ für ein Wasserstoffatom, R⁴ für eine Gruppe -OH und n = für eine ganze Zahl von 1 bis 10 stehen,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(J): Konservierungsmittel-Zusammensetzungen, enthaltend
   a) Ethyllauroylarginat und
   b) 3-(2-Ethylhexyloxy)propan-1,2-diol und
   c) mindestens ein Diol der Formel (III)

      HO-CH₂-CHR³-(CH₂)ₙ-R⁴ (III),

      in der R³ für eine Gruppe -OH, R⁴ für ein Wasserstoffatom und n = für eine ganze Zahl von 1 bis 10 stehen,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(K): Konservierungsmittel-Zusammensetzungen, enthaltend
   a) Ethyllauroylarginat und
   b) 3-(2-Ethylhexyloxy)propan-1,2-diol und
   c) 1,6-Hexandiol,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(L): Konservierungsmittel-Zusammensetzungen, enthaltend
   a) Ethyllauroylarginat und
   b) 3-(2-Ethylhexyloxy)propan-1,2-diol und
   c) 1,2-Hexandiol,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(M): Konservierungsmittel-Zusammensetzungen, enthaltend
   a) mindestens eine Verbindung der Formel (I) 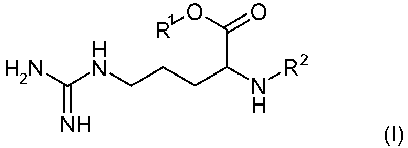 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 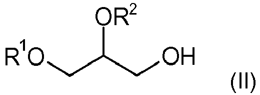 wobei ein Rest der Reste R¹ und R² für einen verzweigten oder linearen, (C₃ bis C₁₂)-Alkylgruppe und der andere dieser Reste für ein Wasserstoffatom steht, und
   c) 2-Phenoxyethanol.
(N): Konservierungsmittel-Zusammensetzungen, enthaltend
   a) Ethyllauroylarginat und
   b) 3-(2-Ethylhexyloxy)propan-1,2-diol und
   c) 2-Phenoxyethanol.
(O): Konservierungsmittel-Zusammensetzungen, enthaltend
   a) mindestens eine Verbindung der Formel (I) 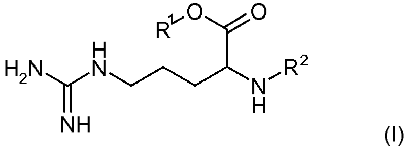 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 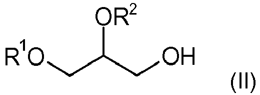 wobei ein Rest der Reste R¹ und R² für einen verzweigten oder linearen, (C₃ bis C₁₂)-Alkylgruppe und der andere dieser Reste für ein Wasserstoffatom steht, und
   c) 2-Phenoxyethanol,
   mit der Massgabe, dass die Verbindungen der Formel (I) und die Verbindungen der Formel (II) in einem Gewichtsverhältnisbereich der Verbindungen der Formel (I) zu Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.
(P): Konservierungsmittel-Zusammensetzungen, enthaltend
   a) Ethyllauroylarginat und
   b) 3-(2-Ethylhexyloxy)propan-1,2-diol und
   c) 2-Phenoxyethanol,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(Q): Konservierungsmittel-Zusammensetzungen, enthaltend
   a) mindestens eine Verbindung der Formel (I) 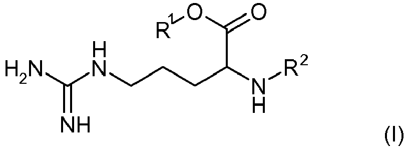 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 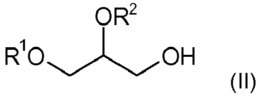 wobei ein Rest der Reste R¹ und R² für einen verzweigten oder linearen, (C₃ bis C₁₂)-Alkylgruppe und der andere dieser Reste für ein Wasserstoffatom steht, und
   c) Natriumsorbat und/oder Kaliumsorbat.
(R): Konservierungsmittel-Zusammensetzungen, enthaltend
   a) Ethyllauroylarginat und
   b) 3-(2-Ethylhexyloxy)propan-1,2-diol und
   c) Natriumsorbat und/oder Kaliumsorbat.
(S): Konservierungsmittel-Zusammensetzungen, enthaltend
   a) mindestens eine Verbindung der Formel (I) 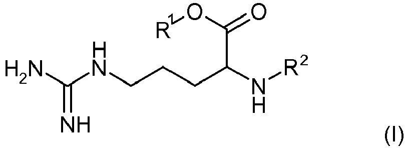 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 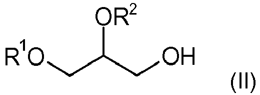 wobei ein Rest der Reste R¹ und R² für einen verzweigten oder linearen, (C₃ bis C₁₂)-Alkylgruppe und der andere dieser Reste für ein Wasserstoffatom steht, und
   c) Natriumsorbat und/oder Kaliumsorbat,
   mit der Massgabe, dass die Verbindungen der Formel (I) und die Verbindungen der Formel (II) in einem Gewichtsverhältnisbereich der Verbindungen der Formel (I) zu Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.
(T): Konservierungsmittel-Zusammensetzungen, enthaltend
   a) Ethyllauroylarginat und
   b) 3-(2-Ethylhexyloxy)propan-1,2-diol und
   c) Natriumsorbat und/oder Kaliumsorbat,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.
(U): Konservierungsmittel-Zusammensetzungen, enthaltend
   a) mindestens eine Verbindung der Formel (I) 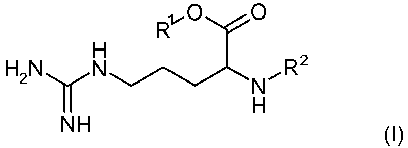 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 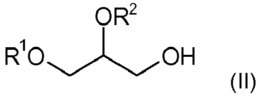 wobei ein Rest der Reste R¹ und R² für einen verzweigten oder linearen, (C₃ bis C₁₂)-Alkylgruppe und der andere dieser Reste für ein Wasserstoffatom steht, und
   c) Benzylalkohol.
(V): Konservierungsmittel-Zusammensetzungen, enthaltend
   c) Ethyllauroylarginat und
   d) 3-(2-Ethylhexyloxy)propan-1,2-diol und
   c) Benzylalkohol.
(W): Konservierungsmittel-Zusammensetzungen, enthaltend
   a) mindestens eine Verbindung der Formel (I) 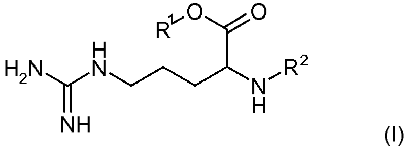 worin
      R¹ eine lineare oder verzweigte (C₁ bis C₁₈)-Alkylgruppe (insbesondere eine (C₁ bis C₃)-alkylgruppe) bedeutet und
      R² für eine lineare oder verzweigte (C₈ bis C₁₄)-Acylgruppe steht, und
   b) mindestens einen Monoglycerylether der Formel (II) 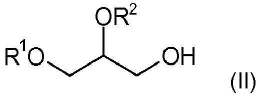 wobei ein Rest der Reste R¹ und R² für einen verzweigten oder linearen, (C₃ bis C₁₂)-Alkylgruppe und der andere dieser Reste für ein Wasserstoffatom steht, und
   c) Benzylalkohol,
   mit der Massgabe, dass die Verbindungen der Formel (I) und die Verbindungen der Formel (II) in einem Gewichtsverhältnisbereich der Verbindungen der Formel (I) zu Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.
(X): Konservierungsmittel-Zusammensetzungen, enthaltend
   a) Ethyllauroylarginat und
   b) 3-(2-Ethylhexyloxy)propan-1,2-diol und
   c) Benzylalkohol,
   mit der Massgabe, dass Ethyllauroylarginat zu 3-(2-Ethylhexyloxy)propan-1,2-diol in einem Gewichtsverhältnisbereich von 1 zu 10 bis 5 zu 1, besonders bevorzugt von 1 zu 6 bis 3 zu 1, ganz besonders bevorzugt von 1 zu 5 bis 1,5 zu 1, enthalten sind.

Die erfindungsgemäßen Konservierungsmittelzusammensetzungen eignen sich insbesondere zur Konservierung von kosmetischen Mitteln und können sowohl in Haut- und Haarbehandlungsmitteln als auch in Mund- und Zahnpflege- und -reinigungsmitteln eingesetzt werden. Ein weiterer Gegenstand der vorliegenden Erfindung ist daher die Verwendung von erfindungsgemäßen Konservierungsmittelzusammensetzungen zur Konservierung kosmetischer Mittel.

Je nach Anwendungsgebiet unterscheidet man bei kosmetischen Mitteln ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- und Mundpflege (Zahn- und Mundpflegemittel, Gebisspflegemittel, Prothesenhaftmittel) und solche zur Haarpflege (Haarwaschmittel, Haarpflegemittel, Haarverfestigungsmittel, Haarverformungsmittel, Mittel zur Farbänderung).

Erfindungsgemäß bevorzugte kosmetische Mittel sind ausgewählt aus der Gruppe der Hautcremes, Hautlotionen, Deodorantien, Antitranspirantien, Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Ein zweiter Erfindungsgegenstand sind kosmetische Mittel, enthaltend in einem kosmetisch akzeptablen Träger die erfindungsgemäße Konservierungsmittelzusammensetzung des ersten Erfindungsgegenstandes (und gegebenenfalls weitere Wirk- und/oder Hilfsstoffe). Dabei sind die Ausführungsformen (A) bis (X) in den erfindungsgemäßen kosmetischen Mitteln bevorzugt enthalten. Üblicherweise werden die erfindungsgemäßen kosmetischen Mittel als Lösungen, Emulsionen oder Suspensionen formuliert, wobei ein wässriger oder ein wässrig-akoholischer Träger oft bevorzugt wird. Erfindungsgemäß besonders bevorzugte kosmetische Mittel enthalten mindestens 30 Gew.-%, bevorzugt mindestens 40 Gew.-%, weiter bevorzugt mindestens 50 Gew.-% und insbesondere mindestens 60 Gew.-% Wasser.

Liegt das kosmetische Mittel in einem wässrig-alkoholischen Träger vor, so enthält dieser Träger bevorzugt Glyzerin. Kosmetische Mittel dieser Ausführungsform enthalten bevorzugt Glyzerin, insbesondere in einer Menge von 0,05 bis 15,0 Gew.-%, besonders bevorzugt von 0,1 bis 10,0 Gew.-%, jeweils bezogen auf das Gesamtgewicht des Mittels.

Die kosmetischen Mittel enthalten die Verbindungen der Formel (I), insbesondere das Ethyllauroylarginat, bezogen auf das Gesamtgewicht des Mittels bevorzugt in einer Menge von 0,005 Gew.-% bis 5,0 Gew.-%, besonders bevorzugt von 0,01 Gew.-% bis 2,5 Gew.-%, ganz besonders bevorzugt von 0,01 Gew.-% bis 2,0, am bevorzugtesten 0,05 bis 1,6 Gew.-%. Diese Einsatzmengen gelten insbesondere für die korrespondierenden Inhaltsstoffe Konservierungsmittelzusammensetzungen der Ausführungsformen (A) bis (X).

Die kosmetischen Mittel enthalten die Verbindungen der Formel (11), insbesondere 3-(2-Ethylhexyloxy)propan-1,2-diol, bezogen auf das Gesamtgewicht des Mittels bevorzugt in einer Menge von 0,001 Gew.-% bis 5,0 Gew.-%, besonders bevorzugt von 0,005 Gew.-% bis 1,5 Gew.-%, ganz besonders bevorzugt von 0,01 Gew.-% bis 0,3 Gew.-%. Diese Einsatzmengen gelten insbesondere für die korrespondierenden Inhaltsstoffe Konservierungsmittelzusammensetzungen der Ausführungsformen (A) bis (X).

Die kosmetischen Mittel enthalten die Diole der Formel (III), insbesondere 1,6-Hexandiol, bezogen auf das Gesamtgewicht des Mittels bevorzugt in einer Menge von 0,01 Gew.-% bis 20,0 Gew.-%, besonders bevorzugt von 0,05 Gew.-% bis 10,0 Gew.-%. Diese Einsatzmengen gelten insbesondere für die korrespondierenden Inhaltsstoffe der in dem erfindungsgemäßen Mittel bevorzugt enthaltenen Konservierungsmittel-zusammensetzungen der Ausführungsformen (A) bis (L).

Die kosmetischen Mittel enthalten die Salze aromatischer Säuren der Formel (IV), bezogen auf das Gesamtgewicht des Mittels bevorzugt in einer Menge von 0,005 Gew.-% bis 0,5 Gew.-%.

Die kosmetischen Mittel enthalten Dehydracetsäure der Formel (Va) und/oder deren Salze der Formel (Vb), bezogen auf das Gesamtgewicht des Mittels bevorzugt in einer Menge von 0,005 Gew.-% bis 0,5 Gew.-%.

Die kosmetischen Mittel enthalten Sorbinsäure und/oder deren Salze (insbesondere Natriumsorbat und/oder Kaliumsorbat) bezogen auf das Gesamtgewicht des Mittels bevorzugt in einer Menge von 0,005 Gew.-% bis 0,5 Gew.-%. Diese Einsatzmengen gelten insbesondere für die korrespondierenden Inhaltsstoffe der in dem erfindungsgemäßen Mittel bevorzugt enthaltenen Konservierungsmittelzusammensetzungen der Ausführungsformen (Q) bis (T).

Die kosmetischen Mittel enthalten 2-Phenoxyethanol, bezogen auf das Gesamtgewicht des Mittels bevorzugt in einer Menge von 0,05 Gew.-% bis 1,0 Gew.-%. Diese Einsatzmengen gelten insbesondere für die korrespondierenden Inhaltsstoffe der in dem erfindungsgemäßen Mittel bevorzugt enthaltenen Konservierungsmittelzusammensetzungen der Ausführungsformen (M) bis (P).

Die kosmetischen Mittel enthalten Benzylalkohol, bezogen auf das Gesamtgewicht des Mittels bevorzugt in einer Menge von 0,05 Gew.-% bis 1,0 Gew.-%. Diese Einsatzmengen gelten insbesondere für die korrespondierenden Inhaltsstoffe der in dem erfindungsgemäßen Mittel bevorzugt enthaltenen Konservierungsmittelzusammensetzungen der Ausführungsformen (U) bis (X).

In einer bevorzugten Ausführungsform enthalten die erfindungsgemäßen kosmetischen Mittel 4-Hydroxybenzoesäure und/oder 4-Hydroxybenzoesäureester in einer Gesamtmenge von null bis maximal 0,1 Gew.-%, bevorzugt null bis 0,05 Gew.-%, besonders bevorzugt null bis 0,00001 Gew.-%, bezogen auf das Gewicht des gesamten kosmetischen Mittels.

Erfindungsgemäß bevorzugte Mittel sind Hautbehandlungsmittel. Vorteilhafterweise liegen erfindungsgemäße Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet). Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Bevorzugte optionale Inhaltsstoffe der erfindungsgemäßen kosmetischen Mittel können über ihre Funktion definiert werden. Natürlich können manche Inhaltsstoffe auch multifunktionell sein. Bevorzugte Inhaltsstoffe der erfindungsgemäßen kosmetischen Mittel können sein: Absorptionsmittel, antimikrobielle Stoffe, Bindemittel, Bleichmittel, Chelatbildner, Emollientien, Emulgatoren/ Dispergatoren, Emulsionsstabilisatoren, Enthaarungsmittel und Wirkstoffe mit Haarwuchs inhibierender Wirkung, Feuchtigkeitsspender, Filmbildner, Farbstoffe, Konservierungsstoffe, Korrosionsschutzmittel, hautkühlende Wirkstoffe, pH-Wert-Regler/ Puffersubstanzen, Tenside/waschaktive Substanzen, Treibgase, Trübungsmittel, UV-Absorber/ Lichtfiltersubstanzen, Vergällungsmittel, Viskositätsregler. Vorgenannte Inhaltsstoffe können gemäß weiterer bevorzugter Ausführungsformen als einziger Zusatz in erfindungsgemäß bevorzugten Mitteln enthalten sein.

Daneben können auch Kombinationen der vorgenannten Inhaltsstoffe in den erfindungsgemäß bevorzugten Mitteln enthalten sein.

Zur stabilen Verdickung der erfindungsgemäßen kosmetischen Mittel eignet sich bevorzugt mindestens ein Verdickungsmittel, ausgewählt aus mindestens einer Verbindung aus der Gruppe, die gebildet wird aus
- Homopolymer oder Copolymer der 2-Methyl-2[(1-oxo-2-propenyl)amino]-1-propansulfonsäure (AMPS),
- Homopolymer oder Copolymer der Acrylsäure und/oder der Methacrylsäure,
- Polysaccharid, bevorzugt Hydroxyethylcellulose, Hydroxypropylcellulose, Hydroxyethylmethylcellulose, Hydroxypropylmethylcellulose, Xanthan Gum, Guar gum,
- Bentonit, gegebenenfalls an der Oberfläche hydrophil oder hydrophob modifiziert.

Die erfindungsgemäßen kosmetischen Mittel enthalten daher bevorzugt zusätzlich mindestens ein Homopolymer oder Copolymer der 2-Methyl-2[(1-oxo-2-propenyl)amino]-1-propansulfonsäure (AMPS), dessen Säuregruppen teilweise oder vollständig neutralisiert sind. Besagtes Polymer ist wiederum bevorzugt vernetzt. Das erfindungsgemäße kosmetische Mittel enthält besagte Polymere bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht des Mittels. Dies gilt insbesondere für erfindungsgemäße Mittel, die die bevorzugten Konservierungsmittelzusammensetzungen der Ausführungsformen (A) bis (X) (insbesondere kombiniert mit den bevorzugten Einsatzmengen deren Inhaltsstoffe im kosmetischen Mittel) enthalten.

Aus der partiellen Neutralisation der Säuregruppen resultieren der neutralisierte Monomerbaustein, beispielsweise Natrium-2-methyl-2[(1-oxo-2-propenyl)amino]-1-propansulfonat oder Ammonium-2-methyl-2[(1-oxo-2-propenyl)amino]-1-propansulfonat, und der nicht-neutralisierte, saure Monomerbaustein, 2-Methyl-2[(1-oxo-2-propenyl)amino]-1-propansulfonsäure. Als neutralisiertes Monomer geeignet sind alle 2-Methyl-2[(1-oxo-2-propenyl)amino]-1-propansulfonatsalze. Bevorzugt sind das Natrium-, Kalium-, Ammonium-, Ethanolamin- oder Aminosäure-Salz. Besonders bevorzugt sind Natrium-2-methyl-2[(1-oxo-2-propenyl)amino]-1-propansulfonat und Ammonium-2-methyl-2[(1-oxo-2-propenyl)amino]-1-propansulfonat.

Ein erstes bevorzugtes Verdickungsmittel ist das vernetzte AMPS-Homopolymer Ammonium Polyacryloyldimethyl Taurate. Ein derartiges vernetztes AMPS-Homopolymer ist beispielsweise von Clariant unter der Bezeichnung Hostacerin AMPS erhältlich.

Besonders bevorzugt sind neben den vernetzten AMPS-Homopolymeren die vernetzten AMPS-Copolymere. Bevorzugte vernetzte AMPS-Copolymere enthalten neben AMPS ein, zwei oder drei, verschiedene Monomere, die neutral sein oder eine schwache Säurefunktion aufweisen können. Selbstverständlich sind auch vernetzte AMPS-Copolymere geeignet, die neben AMPS vier oder sogar mehr Monomere aufweisen.

In einer weiteren bevorzugten Ausführungsform der vorliegenden Erfindung enthält das verdikkende vernetzte AMPS-Copolymer als zweites Monomer ein neutrales Monomer, das ausgewählt ist aus 2-Hydroxyethylacrylat, 2,3-Dihydroxypropylacrylat, 2-Hydroxyethylmethacrylat, 2,3-Dihydroxypropylmethacrylat, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Düsopropylacrylamid, den ethoxylierten Derivaten der vorstehend genannten Ester und Amide, sowie N-Vinylpyrrolidon. Besonders bevorzugt ist das neutrale Polyelektrolytmonomer ausgewählt aus 2-Hydroxyethylacrylat, 2-Hydroxyethylmethacrylat, 2,3-Dihydroxypropylmethacrylat, den ethoxylierten Derivaten der genannten Ester, Acrylamid, Dimethylacrylamid, sowie N-Vinylpyrrolidon. Außerordentlich bevorzugte neutrale Polyelektrolytmonomere sind ausgewählt aus 2-Hydroxyethylacrylat, Acrylamid, Dimethylacrylamid und Vinylpyrrolidon. Außerordentlich bevorzugte vernetzte AMPS-Copolymere sind ausgewählt aus vernetzten Copolymeren, bestehend aus AMPS und N-Vinylpyrrolidon, AMPS und 2-Hydroxyethylacrylat, AMPS und Acrylamid, AMPS und Dimethylacrylamid.

Besonders bevorzugte ethoxylierte Derivate der vorstehend genannten (Meth)Acrylsäureester und (Meth)Acrylsäureamide sind solche der nachstehenden Formel (AMPS-Copol), in denen R1 und R3, die gleich oder verschieden sind, ein Wasserstoffatom oder eine in etwa lineare oder verzweigte Alkylgruppe mit 1 bis 6 Kohlenstoffatomen bedeuten; Y O oder NH bedeutet; R2 eine Kohlenwasserstoffgruppe mit 6 bis 50 Kohlenstoffatomen ist; und x die Molzahl des Alkylenoxids angibt und im Bereich von 1 bis 50, bevorzugt 8 bis 20, liegt:

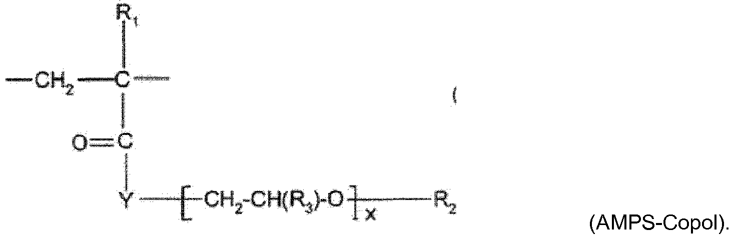

Ein bevorzugtes vernetztes Ammonium Acryloyldimethyltaurate/VP Copolymer ist von Clariant unter der Bezeichnung Aristoflex AVC erhältlich.

In einer weiteren bevorzugten Ausführungsform der vorliegenden Erfindung enthält das verdikkende vernetzte AMPS-Copolymer als zweites Monomer ein Monomer mit schwacher Säurefunktion, das ausgewähl ist aus Acrylsäure, Methacrylsäure, Itaconsäure und Maleinsäure, wobei die schwache Säurefunktion teilweise oder vollständig neutralisiert ist und als Salz, bevorzugt als Natrium-, Kalium-, Ammonium-, Ethanolamin- oder Aminosäure-Salz, besonders bevorzugt als Natriumsalz, vorliegt. Weitere außerordentlich bevorzugte vernetzte AMPS-Copolymere sind ausgewählt aus vernetzten Copolymeren, bestehend aus AMPS und Natriumacrylat, AMPS und Natriummethacrylat, AMPS und Acrylamid und Natriumacrylat, AMPS und Acrylamid und Natriumacrylat und Acrylsäure.

Ein bevorzugtes vernetztes Sodium Acrylate/Sodium Acryloyldimethyl Taurate Copolymer ist von Seppic unter der Bezeichnung Simulgel EPG erhältlich.

Weitere bevorzugte vernetzte Copolymere mit 2-Methyl-2[(1-oxo-2-propenyl)amino]-1-propansulfonsäure (AMPS) sind kommerziell erhältlich, z. B. unter den Handelsnamen Simulgel^{®}NS, Simulgel^{®}I-NS 100, Simulgel^{®}FL, Sepiplus^{®} S, Simulgel^{®}600, Sepiplus 400, Simulgel^{®}SMS 88, Simulgel^{®}EG und Simulgel^{®}EPG von der Firma Seppic.

Die erfindungsgemäßen kosmetischen Mittel enthalten zusätzlich zu den vorgenannten AMPS-Polymeren oder an deren Stelle bevorzugt mindestens ein Homopolymer oder Copolymer der Acrylsäure und/oder Methacrylsäure, dessen Säuregruppen teilweise oder vollständig neutralisiert sind. Besonders bevorzugt sind Homopolymere der Acrylsäure. Solche Polymere besitzen die INCI-Bezeichnung Carbomer.

Das erfindungsgemäße kosmetische Mittel enthält besagte Polymere, insbesondere Homopolymere der Acrylsäure, bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht des Mittels. Dies gilt insbesondere für erfindungsgemäße Mittel, die die bevorzugten Konservierungsmittelzusammensetzungen der Ausführungsformen (A) bis (X) (insbesondere kombiniert mit den bevorzugten Einsatzmengen deren Inhaltsstoffe im kosmetischen Mittel) enthalten.

Die erfindungsgemäßen kosmetischen Mittel enthalten zusätzlich zu den vorgenannten AMPS-Polymeren oder an deren Stelle, zusätzlich zu den Homo-/Copolymeren der Acrylsäure und/oder Methacrylsäure oder an deren Stelle bevorzugt mindestens ein Polysaccharid, bevorzugt ausgewählt aus mindestens einer Verbindung der Gruppe, die gebildet wird aus Xanthan Gum, Guar Gum, Hydroxyethylcellulose, Hydroxypropylcellulose, Hydroxyethylmethylcellulose, Hydroxypropylmethylcellulose.

Das erfindungsgemäße kosmetische Mittel enthält besagte Polysaccharide, insbesondere die bevorzugten Vertreter, bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht des Mittels. Dies gilt insbesondere für erfindungsgemäße Mittel, die die bevorzugten Konservierungsmittelzusammensetzungen der Ausführungsformen (A) bis (X) (insbesondere kombiniert mit den bevorzugten Einsatzmengen deren Inhaltsstoffe im kosmetischen Mittel) enthalten.

Die erfindungsgemäßen Mittel enthalten als Fettkörper besonders bevorzugt mindestens einen Fettstoff. Unter Fettstoffen zu verstehen Fettsäuren, Fettalkohole, natürliche und synthetische Wachse und natürliche und synthetische kosmetische Ölkomponenten zu verstehen.

Die Fettstoffe sind in den erfindungsgemäßen Mitteln bevorzugt in einer Menge von 5,0 bis 50,0 Gew.-%, besonders bevorzugt von 8,0 bis 30,0 Gew.-%, jeweils bezogen auf das Gesamtgewicht des Mittels, enthalten.

Als Fettstoff eignen sich bevorzugt lineare, gesättigte primäre Alkohole mit 14 - 30 Kohlenstoffatomen, Fettsäuren mit 6 bis 30 Kohlenstoffatomen, Triglyzeride, Wachse, Fettsäureester, natürliche Öle, Silikone.

Als eine Hauptkomponenten des Fettkörpers enthalten die erfindungsgemäßen Mittel bevorzugt mindestens einen linearen, gesättigten primären Alkohol mit 14 - 30 Kohlenstoffatomen.

Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol (Arachylalkohol), Behenylalkohol, Lignocerylalkohol, Cerylalkohol und Myricylalkohol und Mischungen hiervon, insbesondere technische Mischungen wie Arachidylalkohol und Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon. Besonders bevorzugt sind Mischungen aus Cetearylalkohol und Behenylalkohol.

Die vorgenannten Alkohole werden im Folgenden auch synonym als "Fettalkohole" bezeichnet.

Bevorzugte kosmetische Mittel enthalten mindestens einen linearen, gesättigten primären Alkohol mit 14 - 30 Kohlenstoffatomen in einer Gesamtmenge von 0,5 bis 10,0 Gew.-%, bevorzugt 1,0 bis 8,0 Gew.-%, jeweils bezogen auf das Gesamtgewicht des Mittels.

Als weitere Fettstoffe können die erfindungsgemäßen Mittel bevorzugt mindestens eine Fettsäure mit 6 bis 30 Kohlenstofatomen enthalten.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge an Fettsäure beträgt dabei bevorzugt 0,1 bis 15 Gew.%, bezogen auf das gesamte Mittel. In einer besonders bevorzugten Ausführungsform beträgt die Menge 0,5 bis 10 Gew.%, wobei ganz besonders vorteilhaft Mengen von 1 bis 5 Gew.% sind.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, eignen sich bevorzugt:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle, insbesondere Capryl-/Caprinsäuretriglyzerid.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin.

Wenn das erfindungsgemäße Mittel zusätzlich als Fettkörper mindestens einen Fettstoff enthält, enthält es wiederum bevorzugt zusätzlich mindestens einen Emulgator.

Emulgatoren sind in den erfindungsgemäßen Mitteln dieser Ausführungsform bevorzugt in einer Menge von 0,25 bis 10,0 Gew.-% enthalten. Als Emulgator kommt bevorzugt mindestens eine Verbindung ausgewählt aus einer oder mehrerer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkyl- sowie Alkenyl-, -mono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter (C₆ bis C₂₂)-Fettsäuren, Ricinolsäure oder 12-Hydroxystearinsäure mit Glycerin, Polyglycerin, Pentaerythrit, Dipenta-erythrit, Zuckeralkoholen (z.B. Sorbit) oder Alkylglucosiden (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Polysiloxan-Polyalkyl-Polyether-Copolymere bzw. entsprechende Derivate;
(11) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin sowie
(12) Lecithin,
(13) N-Acylaminosäuren und/oder N-Acylierte Proteine jeweils mit einem (C₈ bis C₃₀)-Acylrest,
(14) (C₁₂ bis C₂₀)-Alkylphosphat (bevorzugt deren Kaliumsalz) insbesondere in Kombination mit Emulgatoren aus Gruppe (8).

Als besonders bevorzugten Emulgator enthalten bevorzugte erfindungsgemäße Mittel mit besagtem Fettkörper bevorzugt mindestens ein Alkylglycosid mit einem linearen oder verzweigten Alkyl- oder Alkenylrest mit 14 - 30 Kohlenstoffatomen mit der allgemeinen Formel (E4-II) auf,

R⁴O-[G]ₚ (E4-II)

in der R⁴ für einen Alkyl- oder Alkenylrest mit 14 bis 30, bevorzugt 16 bis 22 Kohlenstoffatomen, G für einen Zuckerrest mit 5 oder 6 Kohlenstoffatomen (insbesondere Glucose oder Xylose) und p für Zahlen von 1 bis 10 steht. Diese erfindungsgemäß bevorzugten Mittel umfassen wiederum bevorzugt die Konservierungsstoffzusammensetzungen der bevorzugten Ausführungsformen (A) bis (X) des ersten Erfindungsgegenstandes, insbesondere kombiniert mit den bevorzugten Einsatzmengen deren Inhaltsstoffe im kosmetischen Mittel.

Die Verbindungen der obigen Formel (E4-II) können nach den einschlägigen Verfahren der präparativen organischen Chemie erhalten werden. Die Alkyl- und Alkenyloligoglycoside können sich von Aldosen bzw. Ketosen mit 5 oder 6 Kohlenstoffatomen, wie insbesondere Fructose, Glucose und Xylose, vorzugsweise von Glucose (so genannte APG) oder Xylose (so genannte APX), ableiten, wobei Glucose als Zuckerrest außerordentlich bevorzugt ist. Die bevorzugten Alkyl- und/oder Alkenyloligoglycoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglycosiden, an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 14 - 30, bevorzugt 16 - 22 Kohlenstoffatomen ableiten. Typische Beispiele sind Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische.

Besonders bevorzugt werden die Alkyl- und Alkenyloligoglycoside als Gemisch mit dem entsprechenden, zur Alkylierung eingesetzten Alkoholschnitt eingesetzt. Hierbei handelt es sich um so genannte selbstemulgierende Mischungen aus langkettigen Fettalkoholen und entsprechend alkylierten Glycosiden. Bevorzugt werden Mischungen eingesetzt, die unter den Bezeichnungen Montanov 68, Montanov 202, Montanov L und Montanov 82 von der Firma Seppic erhältlich sind. Diese Mischungen enthalten ca. 15 Gew.-% Alkylglucosid und ca. 85 Gew.-% Fettalkohole. Selbstverständlich können die Alkyl- und Alkenyloligoglycoside aber auch in reiner Form eingesetzt werden, z. B. das Cetearyl Glucoside, erhältlich beispielsweise unter der Bezeichnung Tego Care CG 90. Bevorzugte derartige Mischungen sind (gemäß der INCI-Bezeichnung): C14-22 Alcohols, C12-20 Alkyl Glucoside (Montanov L), Arachidyl Alcohol, Behenyl Alcohol, Arachidyl Glucoside (Montanov 202), Cetearyl Alcohol, Cetearyl Glucoside (Montanov 68, Emulgade PL 68/50), Cetearyl Alcohol, Coco-Glucoside (Montanov 82).

Weitere bevorzugte fettkörperhaltige, kosmetische Mittel enthalten neben der erfindungsgemäßen Konserveirungsmittelzusammensetzung des ersten Erfindungsgegenstandes als Emulgator eine Mischung aus
- 0,2 - 2,0 Gew.-% mindestens eines Salzes eines C₁₂₋₂₀-Alkylphosphats, insbesondere ein Salz von Cetylphosphat, als anionischen ÖI-in-Wasser-Emulgator,
- 0,1 - 1,7 Gew.-% mindestens eines C₁₄₋₂₀-Mono- oder Diacylglycerids, bevorzugt mindestens eines C₁₆₋₁₈-Mono- oder Diacylglycerids, besonders bevorzugt ausgewählt aus gehärteten Palmölglyceriden, besonders lagerstabil formuliert und konserviert werden können.

Diese Mittel umfassen wiederum bevorzugt die Konservierungsstoffzusammensetzungen der bevorzugten Ausführungsformen (A) bis (X) des ersten Erfindungsgegenstandes, insbesondere kombiniert mit den bevorzugten Einsatzmengen deren Inhaltsstoffe im kosmetischen Mittel.

Das mindestens eine Salz eines C₁₂₋₂₀-Alkylphosphats ist in den erfindungsgemäßen Zusammensetzungen in einer Gesamtmenge von 0,2 - 2,0 Gew.-%, bevorzugt 0,3 - 1,8 Gew.-%, besonders bevorzugt 1,0 - 1,5 Gew.-%, jeweils bezogen auf das Gesamtgewicht der Zusammensetzung, enthalten. Bevorzugte erfindungsgemäße Zusammensetzungen enthalten mindestens ein Salz von Cetylphosphat in einer Gesamtmenge von 0,2 - 2,0 Gew.-%, bevorzugt 0,3 - 1,8 Gew.-%, besonders bevorzugt 1,0 - 1,5 Gew.-%, jeweils bezogen auf das Gesamtgewicht der Zusammensetzung. Weitere bevorzugte erfindungsgemäße Zusammensetzungen enthalten eine Mischung aus Dikaliummonocetylphosphat und Kaliumdicetylphosphat in einer Gesamtmenge von 0,2 - 2,0 Gew.-%, bevorzugt 0,3 - 1,8 Gew.-%, besonders bevorzugt 1,0 - 1,5 Gew.-%, jeweils bezogen auf das Gesamtgewicht der Zusammensetzung. Diese bevorzugten Mittel umfassen jeweils wiederum bevorzugt die Konservierungsstoffzusammensetzungen der bevorzugten Ausführungsformen (A) bis (X) des ersten Erfindungsgegenstandes, insbesondere kombiniert mit den bevorzugten Einsatzmengen deren Inhaltsstoffe im kosmetischen Mittel.

Erfindungsgemäß bevorzugte C₁₄₋₂₀-Mono- oder Diacylglyceride, die den zweiten Teil des erfindungsgemäßen O/W-Emulgatorsystems bilden, sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon. Weitere erfindungsgemäß bevorzugte C₁₄₋₂₀-Mono- oder Diacylglyceridmischungen sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride. Diese bevorzugten Mittel umfassen jeweils wiederum bevorzugt die Konservierungsstoffzusammensetzungen der bevorzugten Ausführungsformen (A) bis (X) des ersten Erfindungsgegenstandes, insbesondere kombiniert mit den bevorzugten Einsatzmengen deren Inhaltsstoffe im kosmetischen Mittel.

Das mindestens eine C₁₄₋₂₀-Mono- oder Diacylglycerid ist in den erfindungsgemäßen Zusammensetzungen in einer Gesamtmenge von 0,1 - 1,7 Gew.-%, bevorzugt 0,2 - 1,4 Gew.-%, besonders bevorzugt 0,9 - 1,1 Gew.-%, jeweils bezogen auf das Gesamtgewicht der Zusammensetzung, enthalten.

Bevorzugte erfindungsgemäße Zusammensetzungen enthalten mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon, in einer Gesamtmenge von 0,1 - 1,7 Gew.-%, bevorzugt 0,2 - 1,4 Gew.-%, besonders bevorzugt 0,9 - 1,1 Gew.-%, jeweils bezogen auf das Gesamtgewicht der Zusammensetzung.

Weitere bevorzugte erfindungsgemäße Zusammensetzungen enthalten als C₁₄₋₂₀-Mono- oder Diacylglyceridmischung gehärtete Palmölglyceride in einer Gesamtmenge von 0,1 - 1,7 Gew.-%, bevorzugt 0,2 - 1,4 Gew.-%, besonders bevorzugt 0,9 - 1,1 Gew.-%, jeweils bezogen auf das Gesamtgewicht der Zusammensetzung.

Weitere bevorzugte erfindungsgemäße Zusammensetzungen enthalten die mindestens eine Komponente a)= Salz eines C₁₂₋₂₀-Alkylphosphats und die mindestens eine Komponente b)= C₁₄₋₂₀-Mono- oder Diacylglycerid im Gewichtsverhältnis 2/3 bis 3/2, bevorzugt 1/1 bis 3/2, besonders bevorzugt 6/5 bis 3/2. Diese bevorzugten Mittel umfassen jeweils wiederum bevorzugt die Konservierungsstoffzusammensetzungen der bevorzugten Ausführungsformen (A) bis (X) des ersten Erfindungsgegenstandes, insbesondere kombiniert mit den bevorzugten Einsatzmengen deren Inhaltsstoffe im kosmetischen Mittel.

Eine erfindungsgemäß besonders bevorzugtes O/W-Emulgatorsystem, umfassend ein Gemisch aus Dikaliummonocetylphosphat und Kaliumdicetylphosphat mit gehärteten Palmölglyceriden, ist als Handelsprodukt "Emulsiphos 677660" (INCI: Potassium Cetyl Phosphate, Hydrogenated Palm Glycerides) von der Firma Symrise erhältlich.

Ganz besonders bevorzugte Ausführungsformen der erfindungsgemäßen Mittel sind die Ausführungsformen (M1) bis (M10):
(M1): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 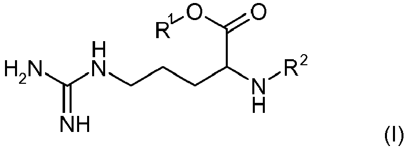 worin
      R¹ eine lineare oder verzweigte (C₁ bis C₆)-Alkylgruppe bedeutet und
      R² für eine lineare oder verzweigte (C₈ bis C₁₄)-Alkylgruppe steht, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 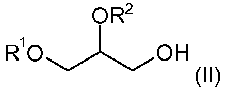 wobei ein Rest der Reste R¹ und R² für eine verzweigte oder lineare (C₃ bis C₁₂)-Alkylgruppe und der andere dieser Reste für ein Wasserstoffatom steht, und
   c) 0,01 Gew.-% bis 20,0 Gew.-%, besonders bevorzugt von 0,05 Gew.-% bis 10,0 Gew.-%, mindestens eines Diols der Formel (III)

      HO-CH₂-CHR³-(CH₂)ₙ-R⁴ (III),

      in der R³ und R⁴ für -H oder -OH und n = für eine ganze Zahl von 1 bis 10 stehen, wobei R³ und R⁴ stets verschieden sind,
   d) 5,0 bis 50,0 Gew.-% mindestens eines Fettstoffs,
   e) 0,25 bis 10,0 Gew.-% mindestens eines Emulgators,
   f) Wasser.
(M2): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 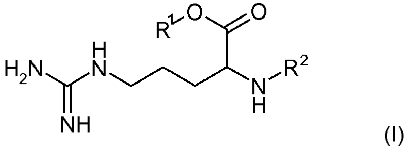 worin
      R¹ eine lineare oder verzweigte (C₁ bis C₆)-Alkylgruppe bedeutet und
      R² für eine lineare oder verzweigte (C₈ bis C₁₄)-Alkylgruppe steht, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 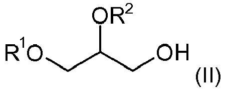 wobei ein Rest der Reste R¹ und R² für eine verzweigte oder lineare (C₃ bis C₁₂)-Alkylgruppe und der andere dieser Reste für ein Wasserstoffatom steht, und
   c) 0,05 Gew.-% bis 1,5 Gew.-%, besonders bevorzugt von 0,05 Gew.-% bis 10,0 Gew.-%, 2-Phenoxyethanol,
   d) 5,0 bis 50,0 Gew.-% mindestens eines Fettstoffs,
   e) 0,25 bis 10,0 Gew.-% mindestens eines Emulgators,
   f) Wasser.
(M3): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 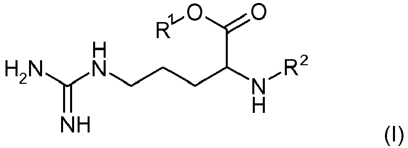 worin
      R¹ eine lineare oder verzweigte (C₁ bis C₆)-Alkylgruppe bedeutet und
      R² für eine lineare oder verzweigte (C₈ bis C₁₄)-Alkylgruppe steht, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 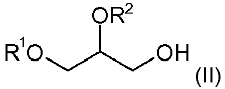 wobei ein Rest der Reste R¹ und R² für eine verzweigte oder lineare (C₃ bis C₁₂)-Alkylgruppe und der andere dieser Reste für ein Wasserstoffatom steht, und
   c) 0,005 Gew.-% bis 0,5 Gew.-%. Natriumsorbat und/oder Kaliumsorbat,
   d) 5,0 bis 50,0 Gew.-% mindestens eines Fettstoffs,
   e) 0,25 bis 10,0 Gew.-% mindestens eines Emulgators,
   f) Wasser.
(M4): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 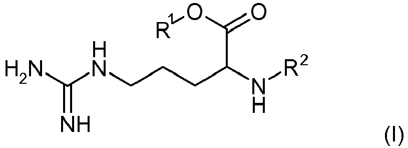 worin
      R¹ eine lineare oder verzweigte (C₁ bis C₆)-Alkylgruppe bedeutet und
      R² für eine lineare oder verzweigte (C₈ bis C₁₄)-Alkylgruppe steht, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 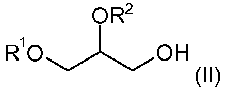 wobei ein Rest der Reste R¹ und R² für eine verzweigte oder lineare (C₃ bis C₁₂)-Alkylgruppe und der andere dieser Reste für ein Wasserstoffatom steht, und
   c) 0,05 Gew.-% bis 0,5 Gew.-%. Benzylalkohol,
   d) 5,0 bis 50,0 Gew.-% mindestens eines Fettstoffs,
   e) 0,25 bis 10,0 Gew.-% mindestens eines Emulgators,
   f) Wasser.
(M5): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 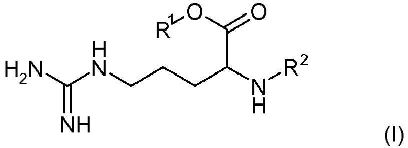 worin
      R¹ eine lineare oder verzweigte (C₁ bis C₆)-Alkylgruppe bedeutet und
      R² für eine lineare oder verzweigte (C₈ bis C₁₄)-Alkylgruppe steht, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 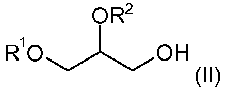 wobei ein Rest der Reste R¹ und R² für eine verzweigte oder lineare (C₃ bis C₁₂)-Alkylgruppe und der andere dieser Reste für ein Wasserstoffatom steht, und
   c) 0,005 Gew.-% bis 0,5 Gew.-%. mindestens eine Salzes aromatischer Säuren der Formel (V) 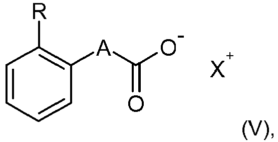 in der R für -H, -OH, ein Halogenatom oder eine C₁₋C₄-Alkylgruppe, A für eine direkte Bindung oder Vinylen und X⁺ für H₃O⁺, ein Alkalimetallion oder den n-ten Teil eines n-wertigen Metallkations steht,
   d) 5,0 bis 50,0 Gew.-% mindestens eines Fettstoffs,
   e) 0,25 bis 10,0 Gew.-% mindestens eines Emulgators,
   f) Wasser.
(M6): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., Ethyllauroylarginat, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., 3-(2-Ethylhexyloxy)propan-1,2-diol, und
   c) 0,01 Gew.-% bis 20,0 Gew.-%, besonders bevorzugt von 0,05 Gew.-% bis 10,0 Gew.-%, mindestens eines Diols der Formel (III)

      HO-CH₂-CHR³-(CH₂)ₙ-R⁴ (III),

      in der R³ und R⁴ für -H oder -OH und n = für eine ganze Zahl von 1 bis 10 stehen, wobei R³ und R⁴ stets verschieden sind,
   d) 5,0 bis 50,0 Gew.-% mindestens eines Fettstoffs,
   e) 0,25 bis 10,0 Gew.-% mindestens eines Emulgators,
   f) Wasser.
(M7): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., Ethyllauroylarginat, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., 3-(2-Ethylhexyloxy)propan-1,2-diol, und
   c) 0,05 Gew.-% bis 1,5 Gew.-%, besonders bevorzugt von 0,05 Gew.-% bis 10,0 Gew.-%, 2-Phenoxyethanol,
   d) 5,0 bis 50,0 Gew.-% mindestens eines Fettstoffs,
   e) 0,25 bis 10,0 Gew.-% mindestens eines Emulgators,
   f) Wasser.
(M8): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., Ethyllauroylarginat, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., 3-(2-Ethylhexyloxy)propan-1,2-diol, und
   c) 0,005 Gew.-% bis 0,5 Gew.-%. Natriumsorbat und/oder Kaliumsorbat,
   d) 5,0 bis 50,0 Gew.-% mindestens eines Fettstoffs,
   e) 0,25 bis 10,0 Gew.-% mindestens eines Emulgators,
   f) Wasser.
(M9): Kosmetisches Mittel enthaltend in einem kosmetisch akzeptablen Träger, bezogen auf sein Gesamtgewicht
   a) 0,005 Gew.-% bis 2,0 Gew.%, ganz besonders bevorzugt 0,05 bis 1,6 Gew.-%., Ethyllauroylarginat, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., 3-(2-Ethylhexyloxy)propan-1,2-diol, und
   c) 0,05 Gew.-% bis 0,5 Gew.-%. Benzylalkohol,
   d) 5,0 bis 50,0 Gew.-% mindestens eines Fettstoffs,
   e) 0,25 bis 10,0 Gew.-% mindestens eines Emulgators,
   f) Wasser.
(M10): Kosmetisches Mittel enthaltend in einem kosmetisch akzeptablen Träger, bezogen auf sein Gesamtgewicht
   a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., Ethyllauroylarginat, und
   b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., 3-(2-Ethylhexyloxy)propan-1,2-diol, und
   c) 0,005 Gew.-% bis 0,5 Gew.-%. mindestens eine Salzes aromatischer Säuren aus der Gruppe Natriumbenzoat, Kaliumbenzoat, Natriumsalicylat, Kaliumsalicylat und Zimtsäure,
   d) 5,0 bis 50,0 Gew.-% mindestens eines Fettstoffs,
   e) 0,25 bis 10,0 Gew.-% mindestens eines Emulgators,
   f) Wasser.

Die erfindungsgemäß bevorzugten Ausführungsformen (M1) bis (M10) enthalten wiederum bevorzugt zusätzlich Glyzerin, insbesondere in einer Menge von 0,05 bis 15,0 Gew.-%, besonders bevorzugt von 0,1 bis 10,0 Gew.-%, jeweils bezogen auf das Gesamtgewicht des Mittels.

Die erfindungsgemäß bevorzugten Ausführungsformen (M1) bis (M10) enthalten wiederum bevorzugt zusätzlich mindestens einen Verdicker, insbesondere aus den vorgenannten bevorzugt geeigneten Verdickungsmitten (*vide supra*). Dabei sind auch die erfindungsgemäß bevorzugten Einsatzmengen wiederum bevorzugt (*vide supra*).

Im Rahmen der oben genannten Ausführungsformen (M1) bis (M10) sind selbstverständlich die bevorzugten Vertreter der Fettstoffe und der Emulgatoren, insbesondere in den vorgenannten Einsatzmengen, bevorzugt (*vide supra*).

### Beispiele:

### 1.0 Wirkungsnachweis

Die Methode ist der PharmEu 6.0/2008 6. Nachtrag (5.1.3; S. 6857 - 6858) entnommen.

### 1.1. Prinzip der Methode

Die Methode wird zur Prüfung der Stabilität von Rohstoffen, Vormischungen und Endprodukten gegen mikrobiellen Verderb eingesetzt. Das zu untersuchende Muster wird künstlich mit Bakterien und Pilzen beimpft (belastet). Das Absterben der Mikroorganismen wird zu definierten Testzeiten quantitativ verfolgt.

### 1.2. Testorganismen

### Standardsatz

| | | |
|---|---|---|
| *Staphylococcus aureus* | ATCC | 6538 |
| *Pseudomonas aeruginosa* | ATCC | 9027 |
| *Candida albicans* | ATCC | 10231 |
| *Aspergillus brasiliensis* | ATCC | 16404 |

Die Stämme werden jeweils einzeln verwendet.

### 1.3. Vortest

Jedes für einen Belastungstest vorgesehene Prüfmuster wurde durch einen Impfausstrich auf TLH-Caso und TLH-Sabouraud zunächst auf aerobe Mikroorganismen geprüft. Dafür wurde eine kleine Menge des zu untersuchenden Produktes mittels einer sterilen Impföse entnommen und auf den o.g. Medien ausgestrichen. Die Medien wurden aerob für 3-7 Tage bei 30°C (TLH-Caso) bzw. 25°C (TLH-Sabouraud) bebrütet.

### 1.4. Vorbereitung der Testorganismen

Alle Bakterien wurden auf Caso-Agar 18-24 Stunden bei 30°C ±2°C vorgezüchtet.
*Candida albicans* wurde 48 h und *A. brasiliensis* bis zu 7 Tage bei 25°C ±2°C auf Sabourraud-Agar vorgezüchtet.

Die gut bewachsenen Testkeimplatten (außer *A. brasiliensis*!) wurden mit einer 0,9%iger NaCl-Lösung (z.B. 10 ml) mit Hilfe eines Glasspatels abgeschwemmt. Die einzelnen Suspensionen wurden durch Glaswolle filtriert und diese mit ca. 10 ml 0,9%iger NaCl-Lösung nachgespült. Es wurde sichergestellt, dass die jeweiligen Einzelsuspensionen in annähernd gleicher Keimzahl von 10⁸ KBE/ml vorliegen. Sie werden durch Verdünnen mit 0,9%iger NaCl-Lösung eingestellt.
Die gut bewachsenen Testkeimplatten von *A. brasiliensis* wurden mit einer 0,9%iger NaCl-Lösung (mit 0,5 g Tween 80/I) (z.B. 10 ml) mit Hilfe eines Glasspatels abgeschwemmt. Die Suspension wurde durch Glaswolle filtriert und mit 0,9%iger NaCl-Lösung (mit 0,5 g Tween 80/I) (z.B. 10 ml) nachgespült. Es wurde sichergestellt, dass die entstehende Sporensuspension in einer Keimzahl von 10⁸ KBE/ml vorliegt. Sie wurde durch Verdünnen mit 0,9%iger NaCl-Lösung (mit 0,5 g Tween 80/I) eingestellt.

Unmittelbar nach Herstellung der Stammsuspension wurde die Keimzahl bestimmt. Diese wurde in den Rohdaten festgehalten. Die Bestimmung der Keimzahl der oben beschriebenen Bakterien- und Pilzsuspension erfolgte gemäß PharmEu 6. Ausgabe 2.6.12 B Harmonisierte Methode im Ausstrichverfahren.

### 1.5. Belastung

30 g ±1g des Prüfmusters wurden in einem sterilen Gefäß einzeln mit 0,3 ml ±0,01 ml der jeweiligen Bakterien bzw. Pilzsuspension beimpft (entspricht jeweils einer 1%igen Beimpfung). Die Vermischung erfolgte mit einem geeigneten Rührstab.
Es wurde darauf geachtet, dass die beimpften Muster atmungsaktiv verschlossen und bei 20-25°C (Raumtemperatur) und im Dunkeln gelagert wurden.

Unmittelbar nach Beimpfung wurde ca. 1g des belasteten Materials in 9 ml Inaktivatorlösung gegeben. Davon wurde eine Impföse und auf die jeweiligen Nährmedien ausgestrichen, um die Beimpfung des Musters zu überprüfen. Die Agarplatten wurden wie unter Punkt 7 beschrieben bebrütet. Die Aussage dieses Austrichs war lediglich qualitativ und konnte in den Rohdaten daher nur mit "+" (Wachstum) oder "-" (kein Wachstum) vermerkt werden.

### 1.6. Quantitative Aufarbeitung

Zu den Testzeiten (siehe Punkte 1.9 und 1.10) wurde mindestens 1g des belasteten Prüfmusters 10%ig mit gepufferter Inaktivatorlösung vermischt. Es wurde darauf geachtet, dass die produkthaltige Inaktivatorlösung vor der Weiterverdünnung in jedem Fall eine homogene Mischung darstellt.

Die Keimzahlbestimmung erfolgte über eine Verdünnungsreihe und Ausplattieren auf TLH-Caso-Agar für Bakterien und auf TLH-Sabouraud für Pilze. Die Bebrütungsparameter entsprach den unter Punkt 1.1 genannten Bedingungen.
Die Bestimmung der Keimzahl erfolgte dabei im Wesentlichen gemäß PharmEu 6. Ausgabe 2.6.12 B Harmonisierte Methode im Ausstrichverfahren. Dabei wurden die belasteten Produkte zuerst 1:10 in Inaktivatorlösung verdünnt und je 0,5 ml dieser Verdünnung auf jeweils 2 Platen TLH-TSA-Agar (für Bakterien) und TLH-Sabouraud-Agar (für Pilze) verteilt. Die Bebrütung erfolgte für die Bakterien bei 30°C +/- 2°C für 3-5 Tage, für die Pilze bei 25°C +/- 2°C für 5-7 Tage.

### 1.7. Inaktivierungsprüfung

Die Inaktivierungsprüfung erfolgte erst nach Auswertung des ersten Prüfwertes. Nur bei den Keimen, die beim ersten Wert kein Wachstum zeigten, wurde eine Inaktivierungsprüfung durchgeführt.
Dafür wurden einer Produktverdünnung (Produkt 1:10 in Inativatorlösung) 1 %ig die zu testenden Keime zugesetzt, so dass eine Belastung von ca. 100KBE/g entstand. Als Kontrolle diente Wasser anstelle von Produkt (=Wasser 1:10 in Inaktivatorlösung). Nach 5 min Inkubationszeit (bei Raumtemperatur) wurde eine Keimzahl dieser Probe ermittelt.
Bei einer erfolgreichen Inaktivierung sollten mindestens 50% der eingesetzten Keime im Vergleich zur Wasserkontrolle wiedergefunden werden.

### 1.8. Testzeiten für Produktprüfungen

Für alle Produkt-Prüfmuster sind als Testzeit der 7., 14. 21. und 28. Tag nach der Belastung vorgesehen.

### 1.9. Auswertung

Es wurde festgehalten, ab welchem Prüftag die Anzahl der Bakterien bzw. der Pilze <10 KBE/g betrug.

| **Konservierungsmittel Gew.-% in Wasser** | | **pH der Probe** | **Prüftag** | **Prüftag** |
|---|---|---|---|---|
| **Name** | **Gew.-%.** | | **Bakterien** | **Pilze** |
| 3-(2-Ethylhexyloxy)-1,2-propandiol | 0.3% | 5.1 | 2 | 7 |
| 1,6-Hexandiol | 6.0% | | | |
| 3-(2-Ethylhexyloxy)-1,2-propandiol | 0.2% | 5.1 | 2 | 2 |
| Ethyl Lauroyl arginat HCl | 0.1% | | | |
| 1,6-Hexandiol | 6.0% | | | |
| Ethyl Lauroyl arginat HCl | 0.1% | 5.1 | 7 | 7 |
| 1,6-Hexandiol | 6.0% | | | |

### 2.0 Beispielformulierungen

### 2.1 Antifaltencreme

| | |
|---|---|
| Ethyl Lauroyl Arginate HCl | 0,20 |
| 3-(2-Ethylhexyloxy)propan-1,2-diol | 0,20 |
| 1,6-Hexandiol | 6,00 |
| Sodium Benzoate | 0,01 |
| 2-Phenoxyethanol | 0,04 |
| Potassium Sorbate | 0,02 |
| Behenyl Alcohol | 2,50 |
| Arachidylalkohol | 2,75 |
| Cetearyl Alcohol | 0,50 |
| Capryl-/Caprinsäure-Triglycerid | 8,00 |
| Dicaprylyl Carbonate | 3,00 |
| Glyceryl Stearate | 1,00 |
| Arachidylglucosid | 0,75 |
| Copernicia Cerifera (Carnauba) Wax | 0,50 |
| Butyrospermum Parkii (Shea) Butter | 1,00 |
| Cocoglycerides | 1,00 |
| Vitamin E Acetate | 0,50 |
| Argania Spinosa Kernel Oil | 3,00 |
| Carthamus Tinctorius (Safflower) Seed Oil | 2,00 |
| Crossential SA14 | 0,50 |
| Rosenwasser | 15,00 |
| 311461 Allplant Essence Organic Laurel | 5,00 |
| Magnesium Chloride | 0,01 |
| Algae Extract | 0,02 |
| Allantoin | 0,20 |
| Taurine | 1,00 |
| Ectoin | 0,10 |
| Betain | 2,00 |
| D-Panthenol | 0,75 |
| Dimethylsilanol Hyaluronate | 0,02 |
| d,l-alpha-Bisabolol | 0,50 |
| Natriumhydroxid | 0,050 |
| Aluminum Starch Octenylsuccinate | 1,00 |
| Carbomer | 0,40 |
| Simulgel EPG | 0,50 |
| Glyzerin | 5,80 |
| Wasser | ad 100 |

### 2.2 Antifaltencreme

| | |
|---|---|
| Ethyl Lauroyl Arginate HCl | 0,20 |
| 3-(2-Ethylhexyloxypropan-1,2-diol | 0,80 |
| 1,6-Hexandiol | 6,00 |
| Butylene Glycol | 0,70 |
| Pentylene Glycol | 0,04 |
| 2-Phenoxyethanol | 0,01 |
| Sodium Benzoate | 0,02 |
| Behenyl Alcohol | 2,50 |
| Arachidyl Alcohol | 2,75 |
| Cetearyl Alcohol | 0,50 |
| Caprylic/Capric Triglyceride | 8,00 |
| Dicaprylyl Carbonate | 3,00 |
| Copernicia Cerifera (Carnauba) Wax | 0,50 |
| Butyrospermum Parkii (Shea) Butter | 1,00 |
| Carthamus Tinctorius (Safflower) Seed Oil | 2,00 |
| Argania Spinosa Kernel Oil | 3,00 |
| Echium Plantagineum Seed Oil | 0,50 |
| Cocoglycerides | 1,00 |
| Arachidyl Glucoside | 0,75 |
| Glyceryl Stearate | 1,00 |
| Tocopheryl Acetate | 0,50 |
| Tocopherol | 0,02 |
| Magnesium Chloride | 0,04 |
| Algae Extract | 0,02 |
| Rosa Damascena Flower Water | 14,00 |
| Allantoin | 0,20 |
| Taurine | 1,00 |
| Ectoin | 0,10 |
| Betaine | 2,00 |
| Panthenol | 0,75 |
| Pantolactone | 0,01 |
| Urea | 0,08 |
| Hydroxyethyl Urea | 1,33 |
| Ammonium Lactate | 0,02 |
| Bisabolol | 0,40 |
| Sodium Lactate | 0,01 |
| Bis-Ethylhexyl Hydroxydimethoxy Benzylmalonate | 0,40 |
| Hydrolyzed Yeast Protein | 0,40 |
| Sodium Hydroxide | 0,03 |
| Sodium Acrylate/Sodium Acryloyldimethyl Taurate Copolymer | 0,18 |
| Aluminum Starch Octenylsuccinate | 0,93 |
| Carbomer | 0,40 |
| Glycerin | 7,50 |
| Wasser | ad 100 |

### 2.3 Antifaltencreme

| | |
|---|---|
| Ethyl Lauroyl Arginate HCl | 0,20 |
| 3-(2-Ethylhexyloxy)propan-1,2-diol | 0,50 |
| 1,6-Hexandiol | 6,00 |
| Butylene Glycol | 0,70 |
| Pentylene Glycol | 0,04 |
| 2-Phenoxyethanol | 0,01 |
| Sodium Benzoate | 0,02 |
| Benzylalcohol | 0,30 |
| Cinnamic Acid | 0,03 |
| Behenyl Alcohol | 2,50 |
| Arachidyl Alcohol | 2,75 |
| Cetearyl Alcohol | 0,50 |
| Caprylic/Capric Triglyceride | 8,00 |
| Dicaprylyl Carbonate | 3,00 |
| Copernicia Cerifera (Carnauba) Wax | 0,50 |
| Butyrospermum Parkii (Shea) Butter | 1,00 |
| Carthamus Tinctorius (Safflower) Seed Oil | 2,00 |
| Argania Spinosa Kernel Oil | 3,00 |
| Echium Plantagineum Seed Oil | 0,50 |
| Cocoglycerides | 1,00 |
| Arachidyl Glucoside | 0,75 |
| Glyceryl Stearate | 1,00 |
| Tocopheryl Acetate | 0,50 |
| Tocopherol | 0,02 |
| Magnesium Chloride | 0,04 |
| Algae Extract | 0,02 |
| Rosa Damascena Flower Water | 14,00 |
| Allantoin | 0,20 |
| Taurine | 1,00 |
| Ectoin | 0,10 |
| Betaine | 2,00 |
| Panthenol | 0,75 |
| Pantolactone | 0,01 |
| Urea | 0,08 |
| Hydroxyethyl Urea | 1,33 |
| Ammonium Lactate | 0,02 |
| Bisabolol | 0,40 |
| Sodium Lactate | 0,01 |
| Bis-Ethylhexyl Hydroxydimethoxy Benzylmalonate | 0,40 |
| Hydrolyzed Yeast Protein | 0,40 |
| Sodium Hydroxide | 0,03 |
| Sodium Acrylate/Sodium Acryloyldimethyl Taurate Copolymer | 0,18 |
| Aluminum Starch Octenylsuccinate | 0,93 |
| Carbomer | 0,40 |
| Glycerin | 7,50 |
| Wasser | ad 100 |

## Patentansprüche

1. Konservierungsmittelzusammensetzung, enthaltend
a) mindestens eine Verbindung der Formel (I) oder deren Salz 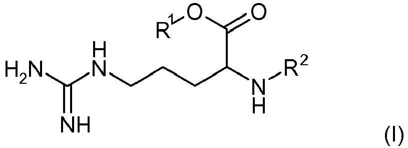 worin
R¹ eine lineare oder verzweigte (C₁ bis C₆)-Alkylgruppe bedeutet und
R² für eine lineare oder verzweigte (C₈ bis C₁₄)-Alkylgruppe steht,
b) mindestens einen Monoglycerylether der Formel (II) 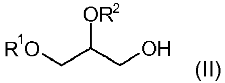 wobei ein Rest der Reste R¹ und R² für eine verzweigte oder lineare (C₃ bis C₁₂)-Alkylgruppe und der andere dieser Reste für ein Wasserstoffatom steht.

2. Konservierungsmittelzusammensetzung nach Anspruch 1, **dadurch gekennzeichnet, dass** die Verbindung der Formel (I) Ethyllauroylarginat ist.

3. Konservierungsmittelzusammensetzung nach einem der vorangehenden Ansprüche, **dadurch gekennzeichnet, dass**. ein Rest der Reste R¹ oder R² des Monoglycerylethers der Formel (II) eine verzweigte oder lineare (C₆ bis C₁₂)-Alkylgruppe, bedeutet und der andere Rest für ein Wasserstoffatom steht.

4. Konservierungsmittelzusammensetzung nach einem der vorangehenden Ansprüche, **dadurch gekennzeichnet, dass** gemäß Formel (II) die besagte Alkylgruppe verzweigt ist.

5. Konservierungsmittelzusammensetzung nach einem der vorangehenden Ansprüche, **dadurch gekennzeichnet, dass** der Monoglycerylether der Formel (II) ausgewählt wird aus mindestens einer Verbindung der Gruppe, die gebildet wird aus 3-(2-Ethylhexyloxy)propan-1,2-diol, 2-(2-Ethylhexyloxy)propan-1,3-diol, 3-Propyloxypropan-1,2-diol, 2-Propyloxypropan-1,3-diol, 3-Octyloxypropan-1,2-diol, 2-Octyloxypropan-1,3-diol, 3-Decyloxypropan-1,2-diol, 2-Decyloxypropan-1,3-diol, 3-Dodecyloxypropan-1,2-diol, 2-Dodecyloxypropan-1,3-diol.

6. Konservierungsmittelzusammensetzung nach einem der vorangehenden Ansprüche, **dadurch gekennzeichnet, dass** der Monoglycerylether der Formel (II) 3-(2-Ethylhexyloxy)propan-1,2-diol ist.

7. Konservierungsmittelzusammensetzung nach einem der vorangehenden Ansprüche, **dadurch gekennzeichnet, dass** das Ethyllauroylarginat und die Verbindungen der Formel (II) in einem Gewichtsverhältnisbereich der Verbindungen der Formel (I) zu Verbindungen der Formel (II) von 1 zu 10 bis 5 zu 1, besonders bevorzugt von 1 zu 6 bis 3 zu 1, ganz besonders bevorzugt von 1 zu 5 bis 1,5 zu 1, enthalten sind.

8. Konservierungsmittelzusammensetzung nach einem der Ansprüche 1 bis 6, **dadurch gekennzeichnet, dass** neben der Verbindung der Formel (I) und dem Monoglycerylether der Formel (II) zusätzlich
c) mindestens eine Verbindung aus der Gruppe, die gebildet wird aus
a. Diolen der Formel (IV)
HO-CH₂-CHR³-(CH₂)ₙR⁴ (IV),
in der R³ und R⁴ für -H oder -OH und n = für eine ganze Zahl von 1 bis 10 stehen, wobei R³ und R⁴ stets verschieden sind,
b. Salzen aromatischer Säuren der Formel (V) 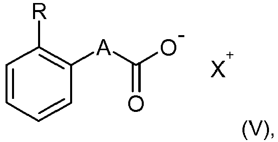 in der R für -H, -OH, ein Halogenatom oder eine C₁-C₄-Alkylgruppe, A für eine direkte Bindung oder eine Vinylengruppe und X⁺ für ein Alkalimetallion oder den n-ten Teil eines n-wertigen Metallkations steht
c. Dehydracetsäure (Va) und/oder deren Salze (Vb) 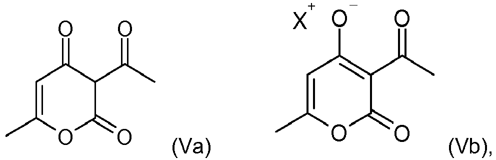 in der X⁺ für ein Alkalimetallion oder den n-ten Teil eines n-wertigen Metallkations steht,
d. Sorbinsäure und deren Salze,
e. 2-Phenoxyethanol,
f. Benzylalkohol,
enthalten ist.

9. Konservierungsmittel-Zusammensetzung nach einem der Ansprüche 1 bis 8, **dadurch gekennzeichnet, dass** 4-Hydroxybenzoesäure und/oder 4-Hydroxybenzoesäureester in einer Gesamtmenge von null bis maximal 0,1 Gew.-%, bevorzugt null bis 0,05 Gew.-%, besonders bevorzugt null bis 0,00001 Gew.-%, bezogen auf das Gewicht der gesamten Konservierungsmittel-Zusammensetzung bzw. des gesamten kosmetischen Mittels enthalten sind.

10. Kosmetisches Mittel umfassend in einem kosmetisch akzeptablen Träger eine Konservierungsmittel-Zusammensetzung der Ansprüche 1 bis 8.

11. Kosmetisches Mittel nach Anspruch 10, **dadurch gekennzeichnet, dass** es bezogen auf sein Gewicht umfasst
a) 0,005 Gew.-% bis 3,0 Gew.-%, bevorzugt von 0,01 Gew.-% bis 2,5 Gew.-%, besonders bevorzugt von 0,01 Gew.-% bis 2,0, ganz besonders bevorzugt 0,05 bis 1,6 Gew.-%., mindestens einer Verbindung der Formel (I) oder deren Salz 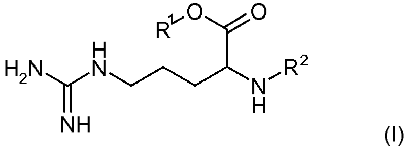 worin
R¹ eine lineare oder verzweigte (C₁ bis C₆)-Alkylgruppe bedeutet und
R² für eine lineare oder verzweigte (C₈ bis C₁₄)-Alkylgruppe steht,
und
b) 0,001 Gew.-% bis 1,0 Gew.-%, bevorzugt von 0,005 Gew.-% bis 0,5 Gew.-%, besonders bevorzugt von 0,01 Gew.-% bis 0,3 Gew.-%., mindestens eines Monoglycerylethers der Formel (II) 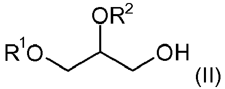 wobei ein Rest der Reste R¹ und R² für eine verzweigte oder lineare (C₃ bis C₁₂)-Alkylgruppe und der andere dieser Reste für ein Wasserstoffatom steht.

12. Kosmetisches Mittel nach einem der Ansprüche 10 oder 11, **dadurch gekennzeichnet, dass** 4-Hydroxybenzoesäure und/oder 4-Hydroxybenzoesäureester in einer Gesamtmenge von null bis maximal 0,1 Gew.-%, bevorzugt null bis 0,05 Gew.-%, besonders bevorzugt null bis 0,00001 Gew.-%, bezogen auf das Gewicht des gesamten kosmetischen Mittels enthalten sind.

## Claims

1. A preservative composition containing
a) at least one compound of formula (I) or a salt thereof 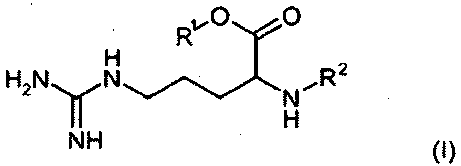 in which
R¹ denotes a linear or branched (C₁ to C₆) alkyl group and
R² represents a linear or branched (C₈ to C₁₄) alkyl group,
b) at least one monoglyceryl ether of formula (II) 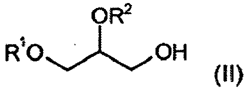 wherein one functional group of the functional groups R¹ and R² represents a branched or linear (C₃ to C₁₂) alkyl group and the other of these functional groups represents a hydrogen atom.

2. The preservative composition according to claim 1, **characterized in that** the compound of formula (I) is ethyl lauroyl arginate.

3. The preservative composition according to one of the preceding claims, **characterized in that** one functional group of the functional groups R¹ or R² of the monoglyceryl ether of formula (II) denotes a branched or linear (C₆ to C₁₂) alkyl group and the other functional group represents a hydrogen atom.

4. The preservative composition according to one of the preceding claims, **characterized in that**, according to formula (II), said alkyl group is branched.

5. The preservative composition according to one of the preceding claims, **characterized in that** the monoglyceryl ether of formula (II) is selected from at least one compound of the group formed by 3-(2-ethylhexyloxy)propane-1,2-diol, 2-(2-ethylhexyloxy)propane-1,3-diol, 3-propyloxypropane-1,2-diol, 2-propyloxypropane-1,3-diol, 3-octyloxypropane-1,2-diol, 2-octyloxypropane-1,3-diol, 3-decyloxypropane-1,2-diol, 2-decyloxypropane-1,3-diol, 3-dodecyloxypropane-1,2-diol, 2-dodecyloxypropane-1,3-diol.

6. The preservative composition according to one of the preceding claims, **characterized in that** the monoglyceryl ether of formula (II) is 3-(2-ethylhexyloxy)propane-1,2-diol.

7. The preservative composition according to one of the preceding claims, **characterized in that** the ethyl lauroyl arginate and the compounds of formula (II) are contained in a weight ratio range of the compounds of formula (I) to compounds of formula (II) of from 1:10 to 5:1, particularly preferably of from 1:6 to 3:1, very particularly preferably of from 1:5 to 1.5:1.

8. The preservative composition according to one of claims 1 to 6, **characterized in that**, apart from the compound of formula (I) and the monoglyceryl ether of formula (II),
c) at least one compound from the group formed by
a. diols of formula (IV)
HO-CH₂-CHR³-(CH₂)ₙ-R⁴ (IV),
in which R³ and R⁴ represent -H or -OH and n represents an integer from 1 to 10, R³ and R⁴ always being different,
b. salts of aromatic acids of formula (V) 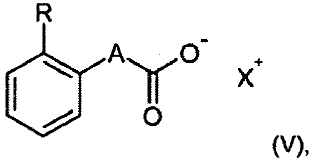 in which R represents -H, -OH, a halogen atom or a C₁-C₄ alkyl group, A represents a direct bond or a vinylene group and X⁺ represents an alkali metal ion or the n-th part of an n-valent metal cation
c. dehydroacetic acid (Va) and/or salts (Vb) thereof 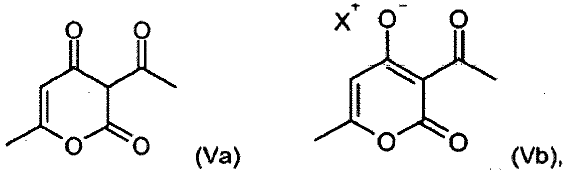 in which X⁺ represents an alkali metal ion or the n-th part of an n-valent metal cation,
d. sorbic acid and salts thereof,
e. 2-phenoxyethanol,
f. benzyl alcohol,
is additionally contained.

9. The preservative composition according to one of claims 1 to 8, **characterized in that** 4-hydroxybenzoic acid and/or 4-hydroxybenzoic acid ester is/are contained in a total amount of from zero to a maximum of 0.1 wt.%, preferably from zero to 0.05 wt.%, particularly preferably from zero to 0.00001 wt.%, based on the weight of the total preservative composition or the total cosmetic agent.

10. A cosmetic agent comprising, in a cosmetically acceptable carrier, a preservative composition from claims 1 to 8.

11. The cosmetic agent according to claim 10, **characterized in that** it comprises, based on its weight,
a) from 0.005 to 3.0 wt.%, preferably from 0.01 to 2.5 wt.%, particularly preferably from 0.01 to 2.0 wt.%, very particularly preferably from 0.05 to 1.6 wt.%, of at least one compound of formula (I) or a salt thereof 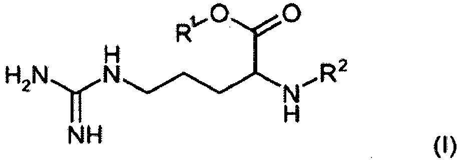 in which
R¹ denotes a linear or branched (C₁ to C₆) alkyl group and
R² represents a linear or branched (C₈ to C₁₄) alkyl group,
and
b) from 0.001 to 1.0 wt.%, preferably from 0.005 to 0.5 wt.%, particularly preferably from 0.01 to 0.3 wt.%, of at least one monoglyceryl ether of formula (II) 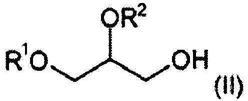 one functional group of the functional groups R¹ and R² representing a branched or linear (C₃ to C₁₂) alkyl group and the other of these functional groups representing a hydrogen atom.

12. The cosmetic agent according to one of claims 10 or 11, **characterized in that** 4-hydroxybenzoic acid and/or 4-hydroxybenzoic acid ester is/are contained in a total amount of from zero to a maximum of 0.1 wt.%, preferably from zero to 0.05 wt.%, particularly preferably from zero to 0.00001 wt.%, based on the weight of the total cosmetic agent.

## Revendications

1. Composition de conservation contenant
a) au moins un composé de la formule (I) ou son sel 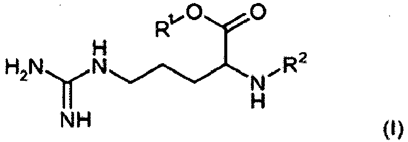 où
R¹ représente un groupe alkyle en C₁ à C₆ linéaire ou ramifié et
R² représente un groupe alkyle en C₈ à C₁₄ linéaire ou ramifié,
b) au moins monoglycéryléther de la formule (II) 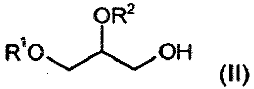 l'un des radicaux R¹ et R² un groupe alkyle en C₃ à C₁₂ linéaire ou ramifié et l'autre de ces radicaux représente un atome d'hydrogène.

2. Composition de conservation selon la revendication 1, **caractérisé en ce que** le composé de la formule (I) l'éthyllauroylarginate.

3. Composition de conservation selon l'une des revendications précédentes, **caractérisée en ce que** l'un des radicaux R¹ ou R² du monoglycéryléther de la formule (II) représente un groupe alkyle en C₆ à C₁₂ ramifiée ou linéaire, et l'autre radical représente un atome d'hydrogène.

4. Composition de conservation selon l'une des revendications précédentes, **caractérisée en ce que**, dans la formule (II), ledit groupe alkyle est ramifié.

5. Composition de conservation selon l'une des revendications précédentes, **caractérisée en ce que** le monoglycéryléther de la formule (II) est choisi parmi au moins un composé du groupe formé par le 3-(2-éthylhexyloxy)propane-1,2-diol, le 2-(2-éthylhexyloxy)propan-1,3-diol, le 3-propyloxypropan-1,2-diol, le 2-propyloxypropan-1,3-diol, le 3-octyloxypropan-1,2-diol, le 2-octyloxypropan-1,3-diol, le 3-décyloxypropan-1,2-diol, le 2-décyloxypropan-1,3-diol, le 3-dodécyloxypropan-1,2-diol, le 2-dodécyloxypropan-1,3-diol.

6. Composition de conservation selon l'une des revendications précédentes, **caractérisée en ce que** le monoglycéryléther de la formule (II) est le 3-(2-éthylhexyloxy)propan-1,2-diol.

7. Composition de conservation selon l'une des revendications précédentes, **caractérisée en ce que** l'éthyllauroylarginate et les composés de la formule (II) sont contenus dans une gamme de rapports en poids des composés de la formule (I) aux composés de la formule (II) allant de 1:10 à 5:1, plus préférablement de 1:6 à 3:1, de manière tout particulièrement préférée de 1:5 à 1,5:1.

8. Composition de conservateur selon l'une des revendications 1 à 6, **caractérisée en ce que**, en plus du composé de la formule (I) et du monoglycéryléther de la formule (II),
c) au moins un composé du groupe formé
a. des diols de la formule (IV)
HO-CH₂-CHR³-(CH₂)ₙ-R⁴ (IV),
où R³ et R⁴ représentent -H ou -OH et n = un nombre entier de 1 à 10, R³ et R⁴ étant toujours différents,
b. des sels d'acides aromatiques de la formule (V) 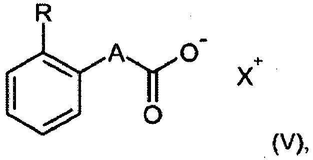 où R représente -H, -OH, un atome d'halogène ou un groupe alkyle en C₁ à C₄, A représente une liaison directe ou un groupe vinylène, et X⁺ représente un ion de métal alcalin ou la n-ième partie d'un cation métallique de valence n
c. l'acide déhydracétique (Va) et/ou leurs sels (Vb) 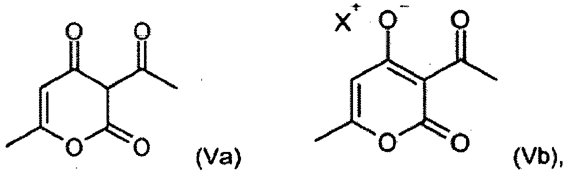 où X⁺ représente un ion de métal alcalin ou la n-ième partie d'un cation métallique de valence n,
d. l'acide sorbique et ses sels,
e. le 2-phénoxyéthanol,
f. l'alcool benzylique.

9. Composition de conservation selon l'une des revendications 1 à 8, **caractérisée en ce que** l'acide 4-hydroxybenzoïque et/ou des esters de l'acide 4-hydroxybenzoïque sont contenus dans une quantité totale allant de zéro à un maximum de 0,1% en poids, de préférence de zéro à 0,05% en poids, de manière particulièrement préférée de zéro à 0,00001% en poids, par rapport au poids de toute la composition de conservation ou de tout l'agent cosmétique.

10. Agent cosmétique comprenant, dans un support cosmétiquement acceptable, une composition de conservation selon les revendications 1 à 8.

11. Composition cosmétique selon la revendication 10, **caractérisée en ce qu'**elle comprend, par rapport à son poids,
a) de 0,005% en poids à 3,0% en poids, de préférence de 0,01% en poids à 2,5% en poids, de manière particulièrement préférée de 0,01% en poids à 2,0% en poids, de manière tout particulièrement préférée de 0,05% en poids à 1,6% en poids, d'au moins un composé de la formule (I) ou de son sel 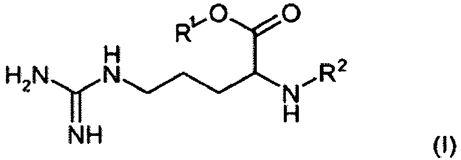 où
R¹ représente un groupe alkyle en C₁ à C₆ linéaire ou ramifié et
R² représente un groupe alkyle en C₈ à C₁₄ linéaire ou ramifié,
et
b) de 0,001% en poids à 1,0% en poids, de préférence de 0,005% en poids à 0,5% en poids, de manière particulièrement préférée de 0,01% en poids à 0,3% en poids, d'au moins un monoglycéryléther de la formule (II) 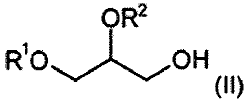 l'un des radicaux R¹ et R² un groupe alkyle en C₃ à C₁₂ linéaire ou ramifié et l'autre de ces radicaux représente un atome d'hydrogène.

12. Agent cosmétique selon l'une des revendications 10 ou 11, **caractérisé en ce que** l'acide 4-hydroxybenzoïque et/ou des esters de l'acide 4-hydroxybenzoïque sont contenus, dans une quantité totale de zéro à un maximum de 0,1% en poids, de préférence de zéro à 0,05% en poids, de manière particulièrement préférée de zéro à 0,00001% en poids, par rapport au poids de tout l'agent cosmétique.
